# EUROPÄISCHE PATENTSCHRIFT

(11) **EP 3 850 357 B1**
(45) Veröffentlichungstag und Bekanntmachung des Hinweises auf die Patenterteilung: **08.11.2023**
(21) Anmeldenummer: 19774051.7
(22) Anmeldetag: 27.08.2019
(51) Int. Cl.: G01N 33/18, C12Q 1/04, G01N 15/06

(54) **AUSTAUSCHBARE KARTUSCHENVORRICHTUNG**
REPLACEABLE CARTRIDGE DEVICE
SYSTÈME DE CARTOUCHE INTERCHANGEABLE

(30) Priorität: 13.09.2018 DE 102018122421
(43) Veröffentlichungstag der Anmeldung: 21.07.2021
(73) Patentinhaber: Bluelab Wasseranalysesysteme GmbH, 72070 Tübingen (DE)
(72) Erfinder: JAUSS, Michael, 72070 Tübingen (DE)
(74) Vertreter: Daub, Thomas
(86) Internationale Anmeldenummer: PCT/EP2019/072771
(87) Internationale Veröffentlichungsnummer: WO 2020/052958

(56) Entgegenhaltungen:
- WO-A1-99/39186
- WO-A1-2016/176169
- AT-B- 409 190
- DE-A1-102014 106 891
- DE-A1-102015 114 956

## Beschreibung

### Stand der Technik

Die Erfindung betrifft eine austauschbare Kartuschenvorrichtung, ein Verfahren zum Betrieb einer austauschbaren Kartuschenvorrichtung und eine Analyseeinrichtung nach dem Oberbegriff der Ansprüche 1, 19 und 20.

Aus der WO 2016/176169 A1, der WO 99/39186 A1, der AT 409 190 B und der DE 10 2015 114956 A1 sind bereits austauschbare Kartuschenvorrichtungen für eine zumindest einer Trinkwasserleitung zugeordnete Messvorrichtung zur Messung einer Trinkwasserkontamination in der Trinkwasserleitung durch Mikroorganismen bekannt. Ferner ist aus der DE 10 2014 106891 A1 bereits ein Probenkarussell bekannt.

Die Aufgabe der Erfindung besteht insbesondere darin, eine gattungsgemäße Vorrichtung, Verfahren und/oder Einrichtung mit verbesserten Eigenschaften hinsichtlich einer Effizienz bereitzustellen. Die Aufgabe wird erfindungsgemäß durch die Merkmale der Patentansprüche 1, 17 und 18 gelöst, während vorteilhafte Ausgestaltungen und Weiterbildungen der Erfindung den Unteransprüchen entnommen werden können.

### Vorteile der Erfindung

Die Erfindung geht aus von einer austauschbaren Kartuschenvorrichtung für eine zumindest einer Trinkwasserleitung insbesondere ortsfest zugeordnete Messvorrichtung zur Messung einer Trinkwasserkontamination in der Trinkwasserleitung durch Mikroorganismen, insbesondere durch Legionellen, mit zumindest einem Kartuschengehäuse, welches mit der Messvorrichtung koppelbar ist, und mit zumindest einer Versuchseinheit, welche zumindest teilweise, insbesondere zumindest zu einem Großteil, in dem Kartuschengehäuse angeordnet ist.

Es wird vorgeschlagen, dass die Versuchseinheit zumindest ein Verbrauchsmaterial aufweist, das zur Durchführung zumindest eines Versuchs zur Messung der Trinkwasserkontamination vorgesehen ist.

Hierdurch kann vorteilhaft eine Effizienz einer Messung einer Trinkwasserkontamination verbessert werden. Insbesondere kann eine Benutzerfreundlichkeit verbessert werden, da Verbrauchsmaterial zur Durchführung des Versuchs zur Messung der Trinkwasserkontamination durch Bereitstellen einer neuen Kartuschenvorrichtung ersetzt werden kann.

Unter einer "Kartuschenvorrichtung" soll insbesondere zumindest eine Konstruktions- und/oder Funktionskomponente und vorzugsweise eine funktionstüchtige Vorrichtung verstanden werden, welche dazu vorgesehen ist, mit zumindest einer Messvorrichtung zur Messung einer Trinkwasserkontamination zu koppeln. Die Kartuschenvorrichtung ist insbesondere Bestandteil einer Analyseeinrichtung, welche zumindest eine solche Kartuschenvorrichtung und/oder Messvorrichtung umfasst. Unter einer "Analyseeinrichtung" soll insbesondere eine Einrichtung verstanden werden, welche zumindest zu einer Prognose und/oder zumindest zu einer Messung einer Trinkwasserkontamination vorgesehen ist. Unter einer "Prognose" soll dabei insbesondere eine Angabe einer Wahrscheinlichkeit einer Trinkwasserkontamination verstanden werden, welche insbesondere anhand von zumindest einer Kontaminationsrisikokenngröße bestimmbar ist, vorzugsweise ohne ein Vorhandensein und/oder eine Anzahl von Mikroorganismen selbst direkt zu bestimmen. Unter einer "Kontaminationsrisikokenngröße" soll insbesondere eine Kenngröße verstanden werden, welche mit einer Trinkwasserkontamination zumindest korreliert ist. Vorzugsweise kann anhand der Kontaminationsrisikokenngröße eine Trinkwasserkontamination zumindest prognostiziert werden. Die Kontaminationsrisikokenngröße ist insbesondere eine Durchflussmenge, eine Temperatur, ein pH-Wert, ein Salzgehalt, ein Metallgehalt, ein Korrosionsgrad, wie beispielsweise ein Korrosionsabbauproduktgehalt, ein Biofilmgehalt, insbesondere eine Dicke eines Biofilms, oder dergleichen.

Unter "vorgesehen" soll insbesondere speziell programmiert, speziell ausgelegt und/oder speziell ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Unter "austauschbar" soll insbesondere durch eine zumindest im Wesentlichen identische neue Kartuschenvorrichtung ersetzbar verstanden werden. Insbesondere ist die Kartuschenvorrichtung werkzeuglos koppelbar. Unter "koppelbar" soll insbesondere kraft- und/oder formschlüssig verbindbar verstanden werden. Unter "kraft- und/oder formschlüssig verbunden" soll dabei insbesondere eine lösbare Verbindung verstanden werden, wobei eine Haltekraft zwischen zwei Bauteilen vorzugsweise durch einen geometrischen Eingriff der Bauteile ineinander und/oder eine Reibkraft zwischen den Bauteilen übertragen wird. Insbesondere weist die Kartuschenvorrichtung zumindest eine Koppeleinheit auf, welche zur Kopplung des Kartuschengehäuses mit der Messvorrichtung vorgesehen ist. Vorzugsweise ist die Koppeleinheit als eine Schnellkopplung ausgebildet. Unter einer "Schnellkopplung" soll insbesondere eine vorzugsweise mechanische und/oder magnetische Einheit verstanden werden, welche dazu vorgesehen ist, zumindest zwei Bauteile werkzeuglos, zerstörungsfrei und/oder wiederholbar miteinander vorzugsweise einhändig zu koppeln, insbesondere ganz besonders vorteilhaft mit einer einzigen Handbewegung eines Bedieners. Beispielsweise kann eine Schnellkopplung einen Klettverschluss, einen Magnetverschluss, einen Klipp- und/oder Rastverschluss, einen Drehverschluss und/oder einen Bajonettverschluss oder dergleichen ausbilden.

Darunter, dass "ein Objekt zumindest einem weiteren Objekt zugeordnet ist", soll insbesondere verstanden werden, dass das Objekt in zumindest einem Betriebszustand und vorzugsweise dauerhaft zu einem Zusammenwirken mit dem weiteren Objekt vorgesehen ist und insbesondere an diesem angeordnet ist. Ferner soll unter einer "ortsfesten Zuordnung" verstanden werden, dass das Objekt mit dem weiteren Objekt fest, insbesondere fluidtechnisch, verbunden und insbesondere an diesem montiert ist.

Unter einer "Trinkwasserleitung" soll insbesondere eine Leitung verstanden werden, welche dazu vorgesehen ist, Trinkwasser, insbesondere zumindest Warmwasser, zu führen. Beispielsweise kann es sich bei der Trinkwasserleitung um eine Leitung eines Trinkwasserhausanschlusses, einer Trinkwasserhauseinführung, einer Trinkwassergarnitur, einer Trinkwasserarmatur, eines Trinkwasserspeichers, insbesondere eines Trinkwasserspeichererhitzers, eines Trinkwasserkreislaufs oder dergleichen handeln. Insbesondere kann eine Leitung einer Trinkwassergarnitur, einer Trinkwasserarmatur, eines Trinkwasserspeichers, insbesondere eines Trinkwasserspeichererhitzers, oder eines Trinkwasserkreislaufs als eine Warmwasser führende Trinkwasserleitung ausgebildet sein. Unter "Warmwasser" soll insbesondere Trinkwasser verstanden werden, welches eine Wassertemperatur von zumindest Raumtemperatur, insbesondere von 20°C aufweist.

Unter einer "Kontamination einer Trinkwasserleitung durch Mikroorganismen" soll insbesondere eine Kontamination der Trinkwasserleitung selbst und/oder von der Trinkwasserleitung geführten Trinkwassers durch Mikroorganismen, insbesondere Legionellen, verstanden werden. Unter einer "Kontamination" soll insbesondere ein Vorkommen von Mikroorganismen verstanden werden, welches eine Anzahl von 10 Mikroorganismen, vorzugsweise 50 Mikroorganismen, und besonders bevorzugt 100 Mikroorganismen je 100 ml Trinkwasser überschreitet. Beispielsweise wird gemäß der deutschen Trinkwasserverordnung (TrinkwV 2001) für Mikroorganismen, bei welchen es sich insbesondere um Legionellen handelt, eine Trinkwasserkontamination bei Überschreitung einer Anzahl von 100 Mikroorganismen je 100 ml des Trinkwassers festgelegt.

Unter einer "Versuchseinheit" soll insbesondere eine Einheit verstanden werden, welche durch Bereitstellung von Versuchsaufbauten zumindest zur Durchführung einer Messung der Trinkwasserkontamination beiträgt. Unter einem "Verbrauchsmaterial" soll insbesondere ein Material verstanden werden, welches nach Gebrauch bei einem Versuch entsorgt werden muss und/oder nicht zu einem weiteren Versuch verwendbar ist.

Unter dem Ausdruck "zumindest zu einem Großteil" soll insbesondere zumindest zu 55 %, vorteilhaft zumindest zu 65 %, vorzugsweise zumindest zu 75 %, besonders bevorzugt zumindest zu 85 % und besonders vorteilhaft zumindest zu 95 % und insbesondere aber auch vollständig verstanden werden.

Ferner wird vorgeschlagen, dass die Versuchseinheit zumindest ein Versuchsband umfasst, welches zumindest ein Versuchsfeld aufweist, welches zur Durchführung des Versuchs vorgesehen ist. Es kann vorteilhaft eine effiziente und flexible Versuchsdurchführung erzielt werden. Insbesondere können mit dem gleichen Versuchsband mehrere Versuche durchgeführt werden, ohne dass dieses ausgetauscht werden muss. Ferner kann eine Verunreinigung und/oder eine Beeinflussung einer Messung vermieden werden. Insbesondere weist das Versuchsband mehrere Versuchsfelder auf. Unter einem "Versuchsfeld" soll insbesondere ein Abschnitt des Versuchsbands verstanden werden, welcher zur Durchführung eines Versuchs, insbesondere eines einzigen Versuchs, vorgesehen ist. Vorzugsweise ist ein Versuchsfeld nach einer Benutzung nicht wiederholt nutzbar. Insbesondere sind die mehreren Versuchsfelder entlang des Versuchsbands voneinander beabstandet angeordnet.

Ferner wird vorgeschlagen, dass in zumindest einem Lagerzustand des Versuchsbands zumindest ein Versuchsbandabschnitt des Versuchsbands zumindest teilweise von zumindest einem weiteren Versuchsbandabschnitt des Versuchsbands bedeckt ist. Es kann vorteilhaft eine Hygiene auf effiziente Art und Weise verbessert werden. Insbesondere kann vermieden werden, dass bereits bei einem Versuch verwendete Versuchsfelder weitere Versuchsfelder kontaminieren. Vorzugsweise ist das Versuchsband zumindest in einem Lagerzustand vor einem Gebrauch bei einer Messung zumindest teilweise von dem weiteren Abschnitt des Versuchsbands bedeckt. Ferner ist insbesondere nach einer Messung der Versuchsabschnitt von dem weiteren Abschnitt des Versuchsbands bedeckt. Insbesondere ist das Versuchsfeld bei einer Durchführung der Messung frei von einer Bedeckung durch einen weiteren Versuchsbandabschnitt des Versuchsbands. Unter "bedeckt" soll insbesondere unmittelbar aufeinander liegend, vorzugsweise aneinander anliegend verstanden werden.

Es ist denkbar, dass zur Bedeckung des Versuchsabschnitts in einem Lagerzustand des Versuchsbands durch zumindest einen weiteren Versuchsbandabschnitt des Versuchsbands, das Versuchsband zumindest teilweise zusammengefaltet ist. Vorzugsweise könnte das Versuchsband zu diesem Zweck leporelloartig, ziehharmonikaartig zusammengefaltet gelagert sein. In einer bevorzugten Ausgestaltung wird vorgeschlagen, dass in zumindest einem Lagerzustand des Versuchsbands das Versuchsband zumindest abschnittsweise aufgerollt gelagert ist. Vorzugsweise ist das Versuchsband zu einer Spule aufgerollt. Insbesondere weist die Versorgungseinheit zumindest zwei Spulen, welche aus aufgewickeltem Versuchsband bestehen, auf, insbesondere eine Abrollspule, auf welcher das Versuchsband zumindest teilweise vor einer Messung gelagert ist, und/oder eine Aufrollspule, auf welcher das Versuchsband zumindest teilweise nach einer Messung gelagert ist. Das Kartuschengehäuse weist insbesondere für jede der Spulen einen separaten Lagerabschnitt auf. Zu einem Antrieb einer Bewegung des Versuchsbands ist dieses vorzugsweise durch ein Abrollen und insbesondere gleichzeitiges Aufrollen der Spulen antreibbar. Insbesondere weisen die Spulen zumindest eine Antriebsachsverbindung auf, über welche diese antreibbar sind.

Ferner wird vorgeschlagen, dass das Versuchsband einen Schichtaufbau, insbesondere in Form einer Sandwichbauweise, aufweist. Es kann vorteilhaft ein effizient aufgebautes Versuchsband bereitgestellt werden. Insbesondere kann eine besonders stabile Bauweise erzielt werden. Weiter vorteilhaft kann eine Herstellung des Versuchsbands vereinfacht werden. Insbesondere können durch einen Schichtaufbau verschieden gestaltete Versuchsabschnitte ausgebildet werden. Unter einem "Schichtaufbau" soll insbesondere ein Aufbau mit zumindest zwei insbesondere voneinander verschieden ausgebildeten Schichten verstanden werden. Unter einer "Sandwichbauweise" soll insbesondere ein Schichtaufbau mit zumindest drei, vorzugsweise zumindest teilweise verschieden voneinander ausgebildeten, Schichten verstanden werden, wobei zumindest eine Schicht zwischen zwei vorzugsweise zumindest im Wesentlichen identisch ausgebildeten Trägerschichten eingeschlossen ist. Unter "zumindest im Wesentlichen identisch" soll insbesondere bis auf Herstellungs- und/oder Montagetoleranzen identisch verstanden werden. Insbesondere weist das Versuchsband zumindest eine Filterschicht auf. Die Filterschicht ist zu einer Filtrierung einer Trinkwasserprobe bei einer Messung vorgesehen. Die Filterschicht ist insbesondere von einem Filtervlies ausgebildet. Die Filterschicht weist insbesondere einen Porendurchmesser von höchstens 0,8 µm, vorzugsweise höchstens 0,6 µm, besonders bevorzugt von höchstens 0,45 µm und ganz besonders bevorzugt von höchstens 0,2 µm auf. Die Filterschicht weist insbesondere eine Schichtdicke von höchstens 0,3 mm, vorzugsweise höchstens 0,2 mm und besonders bevorzugt von höchstens 0,1 mm auf. Die Filterschicht besteht bevorzugt zumindest teilweise und besonders bevorzugt zumindest zu einem Großteil aus einem hygroskopischen Material. Ganz besonders bevorzugt ist die Filterschicht frei von einem hydrophilen Material. Ferner weist das Versuchsband zumindest eine Trägerschicht auf, welche dazu vorgesehen ist, die Filterschicht zu tragen. Die Trägerschicht weist eine Schichtdicke von höchstens 0,9 mm, vorzugsweise höchstens 0,7 mm und besonders bevorzugt von höchstens 0,5 mm auf. Die Trägerschicht besteht vorzugsweise zumindest teilweise und besonders bevorzugt zumindest zu einem Großteil aus einem Kunststoff, wie beispielsweise Polyethylen und/oder Polypropylen. Die Trägerschicht ist insbesondere mit der Filterschicht stoffschlüssig verbunden, vorzugsweise mit dieser verschweißt. Unter "stoffschlüssig verbunden" soll insbesondere verstanden werden, dass Masseteile durch atomare oder molekulare Kräfte zusammengehalten werden, wie beispielsweise beim Löten, Schweißen, Kleben und/oder Vulkanisieren. Ferner weist das Versuchsband zumindest eine weitere Trägerschicht auf. Vorzugsweise ist die weitere Trägerschicht zumindest im Wesentlichen identisch zur Trägerschicht ausgebildet. Die Filterschicht ist zwischen den Trägerschichten eingeschlossen, insbesondere zwischen diesen eingeschweißt. Vorzugsweise weist das Versuchsband zumindest eine Stützschicht auf. Insbesondere ist die Stützschicht zwischen den Trägerschichten angeordnet, insbesondere mit zumindest einer Trägerschicht und mit der Filterschicht verschweißt. Die Stützschicht ist insbesondere dazu vorgesehen, die Filterschicht zu stabilisieren. Vorzugsweise ist die Stützschicht als ein, insbesondere grobmaschiges, Gewebe ausgebildet. Insbesondere weist die Stützschicht eine Schichtdicke zumindest im Wesentlichen analog zu der Schichtdicke der Filterschicht auf.

Ferner wird vorgeschlagen, dass das Versuchsfeld zumindest einen Filter umfasst, welcher zur Abscheidung von Mikroorganismen aus Trinkwasser vorgesehen ist. Es können auf effiziente Art und Weise die Mikroorganismen aus Trinkwasserproben abgeschieden werden. Insbesondere kann durch eine entsprechende Wahl der Porengröße des Filters eine physikalische Auswahl der abzuscheidenden Mikroorganismen getroffen werden. Die Filterschicht bildet zumindest teilweise den Filter des Versuchsfelds aus. Insbesondere bildet die Filterschicht die Filter aller Versuchsfelder des Versuchsbands aus.

Ferner wird vorgeschlagen, dass das Versuchsfeld eine Senke aufweist. Die Senke ist insbesondere zur Aufnahme von Mikroorganismen aus der Trinkwasserprobe vorgesehen. Es kann vorteilhaft auf effiziente Art und Weise ein Zurückhalten der Mikroorganismen innerhalb der Senke des Versuchsfelds erzielt werden. Insbesondere verhindert die Senke ein Verteilen der Mikroorganismen außerhalb des Versuchsfelds auf dem gesamten Versuchsband. Die Senke ist insbesondere durch eine teilweise Ausnehmung des Versuchsfelds gebildet. Vorzugsweise ist die Senke durch ein zumindest teilweises Entfernen der Trägerschicht, insbesondere beider Trägerschichten, ausgebildet. Die Senke legt insbesondere die an der Trägerschicht angeordnete Filterschicht frei. Die Senke ist insbesondere durch bereichsweises Entfernen, insbesondere Ausstanzen, der Trägerschicht im Bereich des Versuchsfelds gebildet. Durch Durchspülen der Filterschicht mit der Trinkwasserprobe werden insbesondere Mikroorganismen an der Filterschicht zurückgehalten, welche sich in der Senke ansammeln.

Ferner wird vorgeschlagen, dass das Versuchsband zumindest ein Reinigungsfeld aufweist, welches zur Spülung mit Trinkwasser vorgesehen ist. Es kann auf effiziente Art und Weise eine Trinkwasserkontamination des Versuchsbands vermieden werden. Insbesondere weist das Reinigungsfeld eine größere Grundfläche auf als das Versuchsfeld. Ferner weist das Versuchsband zumindest ein Trennfeld auf. Das Trennfeld ist insbesondere dazu vorgesehen, das Reinigungsfeld und das Versuchsfeld voneinander zu beabstanden. Das Trennfeld weist einen vollen Schichtaufbau des Versuchsbands auf und ist vorzugsweise frei von Senken oder Ausnehmungen. Ferner ist zumindest das Trennfeld insbesondere dazu vorgesehen, im Lagerzustand das Versuchsfeld zu bedecken.

Ferner wird vorgeschlagen, dass das Reinigungsfeld zumindest eine Ausnehmung aufweist. Es kann vorteilhaft eine effiziente Herstellung erzielt werden. Bei der Ausnehmung handelt es sich insbesondere um eine volle Ausnehmung, welche insbesondere alle Schichten, aus welchen das Versuchsband besteht, betrifft. Insbesondere im Vergleich zum Versuchsfeld sind bei dem Reinigungsfeld die Trägerschicht, die weitere Trägerschicht und die Filterschicht entfernt und/zwar insbesondere ausgestanzt.

Ferner wird vorgeschlagen, dass das Versuchsfeld und das Reinigungsfeld entlang einer Haupterstreckungsrichtung des Versuchsbands versetzt zueinander angeordnet sind. Es kann ein vorteilhaft effizienter Versuchsablauf erzielt werden. Insbesondere kann durch die versetzte Anordnung ein fließbandartiger Ablauf zur Durchführung des Versuchs zur Messung der Trinkwasserkontamination erzielt werden. Unter einer "Haupterstreckungsrichtung" eines Objekts soll dabei insbesondere eine Richtung verstanden werden, welche parallel zu einer längsten Kante eines kleinsten geometrischen Quaders verläuft, welcher das Objekt gerade noch vollständig umschließt. Dabei soll insbesondere eine Haupterstreckung des Versuchsbands in einem abgerollten Zustand des Versuchsbands verstanden werden. Vorzugsweise sind das Versuchsfeld und das Reinigungsfeld in Haupterstreckungsrichtung des Versuchsbands alternierend zueinander angeordnet. Insbesondere ist zwischen einem Versuchsfeld und einem Reinigungsfeld jeweils zumindest ein Trennfeld angeordnet. Ferner ist insbesondere zwischen zumindest zwei zueinander in Haupterstreckungsrichtung versetzt zueinander angeordneten Reinigungsfeldern zumindest ein Trennfeld angeordnet. Das Versuchsfeld, das Trennfeld und das Reinigungsfeld sind dabei in einer sich insbesondere wiederholenden Sequenz in Haupterstreckungsrichtung des Versuchsbands angeordnet. Die Sequenz umfasst insbesondere ein Versuchsfeld, ein Trennfeld, ein Reinigungsfeld, ein weiteres Trennfeld, ein weiteres Reinigungsfeld und ein zusätzliches Trennfeld.

Ferner wird vorgeschlagen, dass die austauschbare Kartuschenvorrichtung zumindest eine Dichteinheit umfasst, welche die Versuchseinheit zumindest abschnittsweise fluiddicht abdichtet. Es kann vorteilhaft auf effiziente Art und Weise eine Hygiene verbessert werden. Insbesondere kann vermieden werden, dass hygienisch reine Versuchsfelder des Versuchsbands kontaminiert werden. Die Dichteinheit weist zumindest eine Dichtung auf, welche die Versuchseinheit, vorzugsweise das Versuchsband der Versuchseinheit, zumindest abschnittsweise fluiddicht abdichtet. Insbesondere kann die Dichteinheit zumindest zwei, vorzugsweise mehrere Dichtungen aufweisen, welche entlang der Haupterstreckungsrichtung des Versuchsbands versetzt zueinander angeordnet sind. Die Dichteinheit dichtet insbesondere einen unbedeckten Versuchsbandabschnitt des Versuchsbands ab. Die Dichteinheit ist insbesondere zwischen der Abrollspule und der Aufrollspule angeordnet.

Ferner wird vorgeschlagen, dass die Dichteinheit dichtend an zumindest einem Versuchsbandabschnitt des Versuchsbands anliegt. Besonders vorteilhaft kann durch ein Zusammenspiel der Senke eines Versuchsfelds und der abdichtenden Dichteinheit ein Verteilen der Mikroorganismen auf einem bei einem Versuch verwendeten Versuchsfeld vermieden werden. Die Dichtung kontaktiert das Versuchsband unmittelbar, wobei insbesondere lediglich die Senke des Versuchsfelds nicht in Kontakt mit der Dichtung steht. Vorzugsweise ist die Dichtung als eine Dichtlippe ausgebildet.

Erfindungsgemäß wird vorgeschlagen, dass die Versuchseinheit zumindest eine Versorgungseinheit zur Bereitstellung von Markern zur Markierung der Mikroorganismen umfasst. Es kann vorteilhaft eine effiziente quantitative Bestimmung der Trinkwasserkontamination erzielt werden. Unter "Markern" sollen insbesondere Farbstoffe, insbesondere fluoreszierende Farbstoffe, verstanden werden, welche dazu vorgesehen sind, sich mit Mikroorganismen, insbesondere einer speziellen Mikroorganismenart, insbesondere Legionellen, Kolibakterien o. dgl., zu koppeln. Die Versorgungseinheit weist insbesondere eine Speichereinheit auf, in welcher die Marker gelagert sind. Die Marker sind insbesondere Verbrauchsmaterial, welches ausgetauscht oder nachgefüllt werden kann. Beispielsweise kann eine Speichereinheit durch eine neue mit neuen Markern gefüllte Speichereinheit ersetzbar sein. Ferner ist denkbar, dass die Speichereinheit mit neuen Markern nachfüllbar ist. Die Versorgungseinheit ist insbesondere zumindest teilweise an dem Kartuschengehäuse angeordnet. Vorzugsweise ist zumindest die Speichereinheit der Versorgungseinheit an dem Kartuschengehäuse angeordnet.

Erfindungsgemäß wird vorgeschlagen, dass die Versorgungseinheit zumindest eine Mikrodosiereinheit umfasst. Hierdurch kann vorteilhaft eine effiziente Dosierung der Marker erfolgen. Vorzugsweise kann eine Überdosierung und Verschwendung der Marker bei einem Versuch vermieden werden. Vorzugsweise kann vermieden werden, dass eine Überdosierung von Markern ein Messergebnis beeinflusst. Unter einer "Mikrodosiereinheit" soll insbesondere eine Einheit verstanden werden, welche zur Dosierung einer Mikrodosis vorgesehen ist. Unter einer "Mikrodosis", soll insbesondere eine Dosis eines Mediums von unter 10 ml, bevorzugt von unter 5 ml und besonders bevorzugt von unter 2 ml verstanden werden. Die Mikrodosiereinheit umfasst insbesondere zumindest eine Mikropumpe, welche zur Förderung einer Mikrodosis vorgesehen ist. Die Mikrodosis umfasst insbesondere zumindest die Marker zur Markierung der Mikroorganismen. Die Mikrodosiereinheit ist insbesondere an dem Kartuschengehäuse angeordnet.

Ferner wird vorgeschlagen, dass die Versorgungseinheit zumindest eine Versorgungsleitung umfasst, welche zur Aufbringung der Marker vorgesehen ist. Die Versorgungsleitung ist fluidtechnisch mit der Speichereinheit und/oder der Mikrodosiereinheit verbunden. Hierdurch kann eine effiziente und insbesondere gezielte Aufbringung der Marker auf ein Versuchsband erzielt werden. Die Versorgungsleitung ist vorzugsweise an dem Kartuschengehäuse angeordnet.

Ferner wird vorgeschlagen, dass die Versuchseinheit zumindest eine Spüleinheit umfasst, welche zum Spülen zumindest eines Verbrauchsmaterials der Versuchseinheit vorgesehen ist. Die Spüleinheit ist insbesondere zu einem Spülen mit Luft vorgesehen. Hierdurch kann eine Effizienz einer Messung verbessert werden. Insbesondere können mittels der Spüleinheit überschüssige Marker vom Versuchsband entfernt werden. Die Spüleinheit umfasst zumindest eine Mikrodosiereinheit. Die Mikrodosiereinheit ist insbesondere zu Förderung von Umgebungsluft vorgesehen, welche zum Spülen vorgesehen ist. Ferner ist denkbar, dass die Spüleinheit zumindest eine Speichereinheit umfasst, in welcher insbesondere destilliertes Wasser gespeichert ist, welches zu einem Spülvorgang genutzt werden kann.

Ferner wird vorgeschlagen, dass die Versuchseinheit zumindest einen Versorgungsanschluss aufweist, welcher an einem abgerollten Abschnitt des Versuchsbands angeordnet ist.

Ferner wird vorgeschlagen, dass die Versorgungseinheit zumindest eine Versorgungsleitung aufweist, welche entlang einer Haupterstreckungsrichtung des Versuchsbands versetzt neben dem Versorgungsanschluss angeordnet ist.

Ferner wird ein Verfahren zum Betrieb einer austauschbaren Kartuschenvorrichtung, insbesondere der zuvor genannten Kartuschenvorrichtung, für eine zumindest einer Trinkwasserleitung insbesondere ortsfest zugeordnete Messvorrichtung zur Messung einer Trinkwasserkontamination in der Trinkwasserleitung durch Mikroorganismen vorgeschlagen, mit zumindest einem Kartuschengehäuse, in welchem zumindest teilweise zumindest eine Versuchseinheit angeordnet ist, welche zumindest ein Verbrauchsmaterial aufweist, das zur Durchführung zumindest eines Versuchs zur Messung der Trinkwasserkontamination vorgesehen ist, wobei in zumindest einem Verfahrensschritt das Kartuschengehäuse mit einer Messvorrichtung gekoppelt wird.

Ferner wird eine Analyseeinrichtung vorgeschlagen, welche zumindest eine zumindest einer Trinkwasserleitung insbesondere ortsfest zugeordnete Messvorrichtung zur Messung einer Trinkwasserkontamination in der Trinkwasserleitung durch Mikroorganismen und zumindest eine austauschbare Kartuschenvorrichtung umfasst.

Die erfindungsgemäße austauschbare Kartuschenvorrichtung, das erfindungsgemäße Verfahren und/oder die erfindungsgemäße Analyseeinrichtung sollen/soll hierbei nicht auf die oben beschriebene Anwendung und Ausführungsform beschränkt sein. Insbesondere können/kann die erfindungsgemäße austauschbare Kartuschenvorrichtung, das erfindungsgemäße Verfahren und/oder die erfindungsgemäße Analyseeinrichtung zu einer Erfüllung einer hierin beschriebenen Funktionsweise eine von einer hierin genannten Anzahl von einzelnen Elementen, Bauteilen und Einheiten sowie Verfahrensschritten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

### Zeichnungen

Weitere Vorteile ergeben sich aus der folgenden Zeichnungsbeschreibung. In den Zeichnungen ist ein Ausführungsbeispiel der Erfindung dargestellt.

Es zeigen:
- Fig. 1: eine Analyseeinrichtung in einer schematischen perspektivischen Darstellung,
- Fig. 2: einen Teil der Analyseeinrichtung mit einer Probenentnahmevorrichtung, einer Messvorrichtung und einer Kartuschenvorrichtung in einer Explosionsdarstellung,
- Fig. 3: einen Teil der Analyseeinrichtung mit einer Probenentnahmevorrichtung,
- Fig. 4: ein Diagramm eines beispielhaften Kontaminationskennfelds zur Prognose einer Trinkwasserkontamination,
- Fig. 5: einen Teil der Kartuschenvorrichtung mit einem Kartuschengehäuse in einer schematischen perspektivischen Darstellung,
- Fig. 6: einen Teil der Kartuschenvorrichtung ohne das Kartuschengehäuse in einer schematischen perspektivischen Darstellung,
- Fig. 7: einen Teil der Kartuschenvorrichtung mit einem Versuchsband in einer schematischen perspektivischen Darstellung,
- Fig. 8: einen Teil der Kartuschenvorrichtung, der Probenentnahmevorrichtung und der Messvorrichtung in einer schematischen perspektivischen Darstellung und
- Fig. 9: einen schematischen Ablaufplan eines beispielhaften Verfahrens zum Betrieb der Analyseeinrichtung.

### Beschreibung des Ausführungsbeispiels

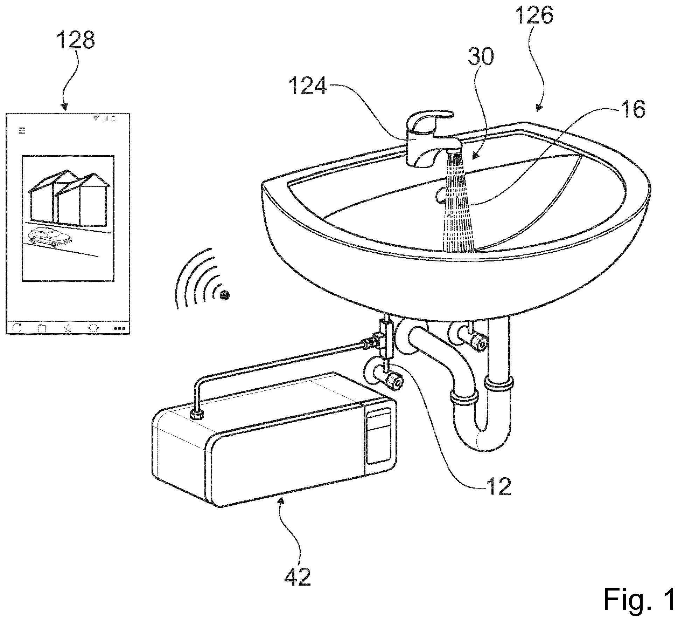

Fig. 1 zeigt eine schematische perspektivische Darstellung einer Analyseeinrichtung 42 und einer Trinkwasserleitung 12. Die Analyseeinrichtung 42 ist einer Trinkwasserleitung 12 zugeordnet. Im vorliegenden Fall ist die Analyseeinrichtung 42 der Trinkwasserleitung 12 ortsfest zugeordnet. Die Analyseeinrichtung 42 ist fest mit der Trinkwasserleitung 12 verbunden. Die Analyseeinrichtung 42 ist zu einer Analyse einer Trinkwasserkontamination der Trinkwasserleitung 12 durch Mikroorganismen 16 vorgesehen. Der Übersichtlichkeit halber sind die Mikroorganismen 16 in Fig. 1 überzeichnet dargestellt. Alternativ könnte die Analyseeinrichtung 42 auch beweglich sein, um diese insbesondere Trinkwasserleitungen 12 an verschiedenen Orten zuzuordnen. Ferner ist denkbar, dass die Analyseeinrichtung 42 mehreren Trinkwasserleitungen 12 zugeordnet sein kann. Vorzugsweise ist die Analyseeinrichtung 42 einer letzten Entnahmestelle eines Trinkwasserleitungssystems zugeordnet, also insbesondere unmittelbar vor einem Endverbraucher.

Die Trinkwasserleitung 12 führt Trinkwasser 30. Im vorliegenden Fall führt die Trinkwasserleitung 12 Warmwasser. Bei der Trinkwasserleitung 12 handelt es sich um eine Warmwasserleitung. Die Trinkwasserleitung 12 ist mit einer Armatur 124 verbunden. Die Armatur 124 ist Teil einer Waschbeckengarnitur 126. Alternativ kann es sich bei der Trinkwasserleitung 12 um eine Trinkwasser 30 führende Leitung eines Trinkwasserhausanschlusses, einer Trinkwasserhauseinführung, eines Trinkwasserspeichers, insbesondere eines Trinkwasserspeichererhitzers, oder dergleichen handeln.

Die Analyseeinrichtung 42 umfasst zumindest eine externe Einheit 128. Die externe Einheit 128 ist von weiteren Komponenten der Analyseeinrichtung 42 separat ausgebildet. Die externe Einheit 128 ist mit weiteren Komponenten der Analyseeinrichtung 42 zu einem Datenaustausch vorgesehen. Die externe Einheit 128 ist zumindest zur Anzeige von Analysedaten vorgesehen. Ferner ist die externe Einheit 128 zu einer Ansteuerung, insbesondere einer Fernsteuerung, der Analyseeinrichtung 42 vorgesehen. Im vorliegenden Fall ist die externe Einheit 128 als ein Smartphone ausgebildet. Alternativ könnte die externe Einheit 128 auch als ein anderes Gerät, insbesondere ein Handgerät, ausgebildet sein, wie beispielsweise ein Tablet, eine Smartwatch, ein Laptop oder dergleichen. Ferner könnte es sich bei der externen Einheit 128 um einen Server, insbesondere einen Cloud-Server, handeln, wie beispielsweise einen Server einer Firmenzentrale.

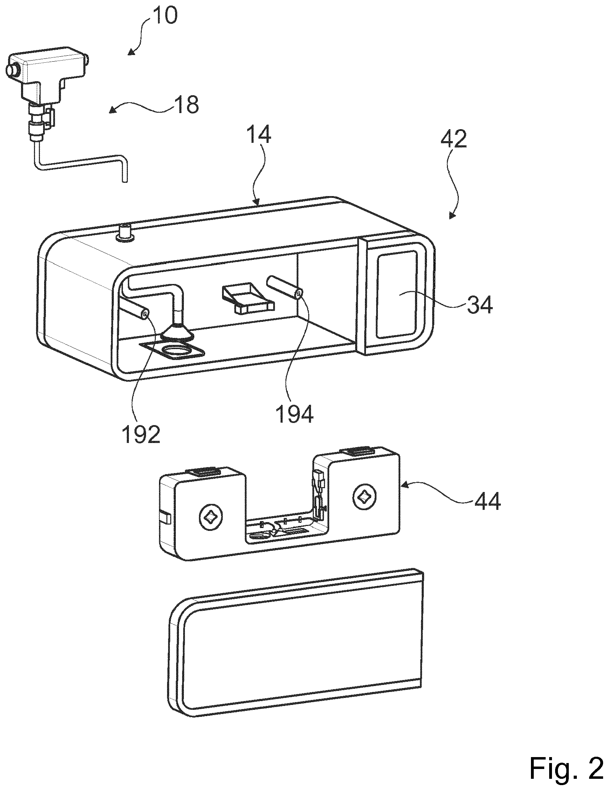

Fig. 2 zeigt einen Teil der Analyseeinrichtung 42 in einer Explosionsdarstellung. Die Analyseeinrichtung 42 weist zumindest eine Messvorrichtung 14 auf. Ferner weist die Analyseeinrichtung 42 zumindest eine Probenentnahmevorrichtung 10 auf. Die Probenentnahmevorrichtung 10 ist dazu vorgesehen, der Messvorrichtung 14 zumindest eine Trinkwasserprobe zur Verfügung zu stellen. Die Probenentnahmevorrichtung 10 ist an der Messvorrichtung 14 angeordnet. Unter einer "Probenentnahmevorrichtung" 10 soll insbesondere zumindest eine Konstruktions- und/oder Funktionskomponente und vorzugsweise eine funktionstüchtige Vorrichtung verstanden werden, welche dazu vorgesehen ist, zumindest einer der Probenentnahmevorrichtung 10 insbesondere ortsfest zugeordneten Trinkwasserleitung 12 zumindest eine Trinkwasserprobe zu entnehmen. Ferner weist die Analyseeinrichtung 42 zumindest eine Kartuschenvorrichtung 44 auf. Die Kartuschenvorrichtung 44 ist an der Messvorrichtung 14 angeordnet. Die Messvorrichtung 14 umfasst zumindest ein Aufnahmegehäuse. Das Aufnahmegehäuse ist dazu vorgesehen, die Kartuschenvorrichtung 44 zumindest teilweise aufzunehmen.

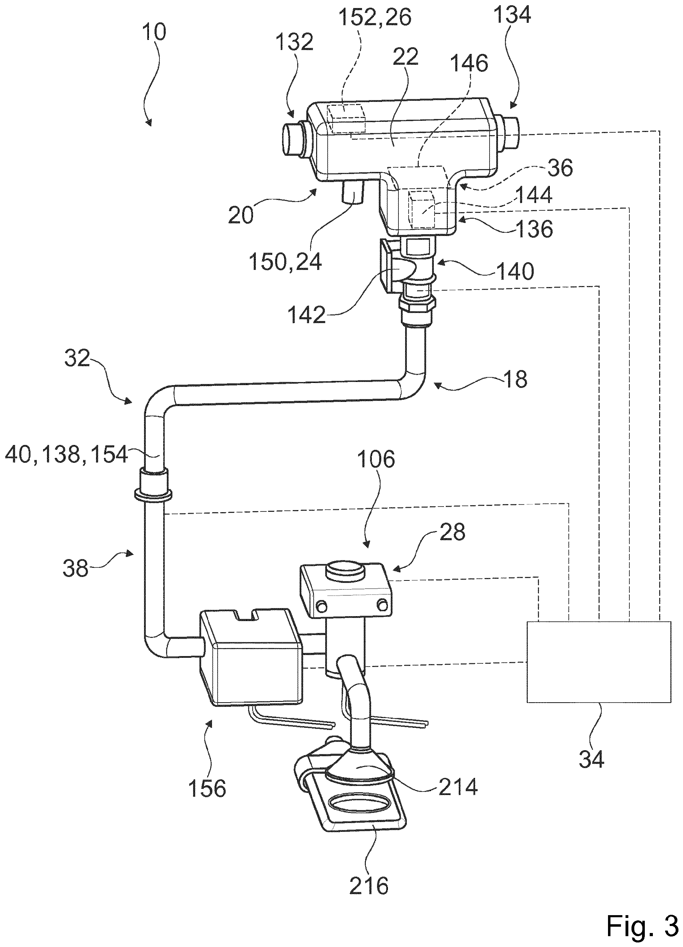

Fig. 3 zeigt einen Teil der Probenentnahmevorrichtung 10 in einer schematischen perspektivischen Darstellung. Die Probenentnahmevorrichtung 10 ist zumindest zum Überwachen einer prognostizierten Trinkwasserkontamination vorgesehen. Zusätzlich ist die Probenentnahmevorrichtung 10 zu einer Entnahme wenigstens einer Trinkwasserprobe der Trinkwasserleitung 12 vorgesehen (vgl. Fig. 1). Die Probenentnahmevorrichtung 10 ist der Trinkwasserleitung 12 zugeordnet. Im vorliegenden Fall ist die Probenentnahmevorrichtung 10 ortsfest der Trinkwasserleitung 12 zugeordnet. Die Probenentnahmevorrichtung 10 ist fest mit der Trinkwasserleitung 12 verbunden.

Die Probenentnahmevorrichtung 10 weist zumindest eine Entnahmeeinheit 18 auf. Die Entnahmeeinheit 18 ist zu einer Trinkwasserentnahme vorgesehen. Die Entnahmeeinheit 18 ist dazu vorgesehen, zumindest einen Teil eines von der Trinkwasserleitung 12 geführten Trinkwassers 30 als eine Trinkwasserprobe der Trinkwasserleitung 12 zu entnehmen. Im vorliegenden Fall bildet die Entnahmeeinheit 18 einen Abzweig der Trinkwasserleitung 12 aus. Ferner könnte die Entnahmeeinheit 18 zumindest teilweise die Trinkwasserleitung 12 und/oder einen Bypass der Trinkwasserleitung 12 ausbilden.

Die Entnahmeeinheit 18 weist zumindest einen Anschluss 132 auf. Der Anschluss 132 ist als Trinkwassereinlass vorgesehen. Der Anschluss 132 ist mit der Trinkwasserleitung 12 verbunden. Ferner weist die Entnahmeeinheit 18 zumindest einen zusätzlichen Anschluss 134 auf. Der zusätzliche Anschluss 134 ist als ein Trinkwasserauslass vorgesehen. Der Anschluss 132 und der zusätzliche Anschluss 134 sind fluidtechnisch einander gegenüberliegend angeordnet.

Ferner weist die Entnahmeeinheit 18 zumindest einen weiteren Anschluss 136 auf. Der weitere Anschluss 136 ist zu der Trinkwasserprobenentnahme vorgesehen. Der weitere Anschluss 136 ist insbesondere zumindest im Wesentlichen quer zu einer Hauptströmungsrichtung des Trinkwassers 30 durch die Trinkwasserleitung 12 angeordnet. Unter "zumindest im Wesentlichen quer" soll insbesondere von zumindest im Wesentlichen parallel verschieden und vorzugsweise zumindest im Wesentlichen senkrecht verstanden werden. Unter "zumindest im Wesentlichen parallel" soll hier insbesondere eine Ausrichtung einer Richtung relativ zu einer Bezugsrichtung, insbesondere in einer Ebene, verstanden werden, wobei die Richtung und die Bezugsrichtung einen Winkel von 0°, insbesondere unter Berücksichtigung einer maximalen Abweichung von kleiner als 8°, vorteilhaft von kleiner als 5° und besonders vorteilhaft von kleiner als 2° einschließen. Unter "zumindest im Wesentlichen senkrecht" soll hier insbesondere eine Ausrichtung einer Richtung relativ zu einer Bezugsrichtung, insbesondere in einer Ebene, verstanden werden, wobei die Richtung und die Bezugsrichtung einen Winkel von 90°, insbesondere unter Berücksichtigung einer maximalen Abweichung von kleiner als 8°, vorteilhaft von kleiner als 5° und besonders vorteilhaft von kleiner als 2° einschließen.

Die Entnahmeeinheit 18 weist zumindest einen Anschlusskopf 22 auf. Der Anschlusskopf 22 ist als ein Dreiwege-Anschlusskopf ausgebildet. Der Anschlusskopf 22 ist T-förmig ausgebildet. Der Anschlusskopf 22 ist zum Anschluss an die Trinkwasserleitung 12 vorgesehen. Der Anschlusskopf 22 stellt den Anschluss 132 bereit. Ferner stellt der Anschlusskopf 22 den zusätzlichen Anschluss 134 bereit. Zudem stellt der Anschlusskopf 22 den weiteren Anschluss 136 bereit.

Die Probenentnahmevorrichtung 10 weist zumindest eine Sensoreinheit 20 auf. Die Sensoreinheit 20 ist zur Sensierung zumindest einer Kontaminationsrisikokenngröße vorgesehen. Die Kontaminationsrisikokenngröße ist eine Temperatur des Trinkwassers 30. Im vorliegenden Fall ist die Sensoreinheit 20 zu einer Sensierung zumindest einer weiteren Kontaminationsrisikokenngröße vorgesehen. Die weitere Kontaminationsrisikokenngröße ist von der Kontaminationsrisikokenngröße verschieden. Die weitere Kontaminationsrisikokenngröße ist ein Durchfluss des Trinkwassers 30.

Die Sensoreinheit 20 ist zumindest teilweise in der Entnahmeeinheit 18 angeordnet. Darunter, dass "die Sensoreinheit 20 zumindest teilweise in der Entnahmeeinheit 18 angeordnet ist", soll insbesondere verstanden werden, dass zumindest ein Sensor 150, 152 der Sensoreinheit 20 und vorzugsweise alle Sensoren 150, 152 der Sensoreinheit 20 in der Entnahmeeinheit 18 angeordnet sind. Ferner soll unter "in etwas angeordnet" sein insbesondere auch in etwas integriert sein verstanden werden. Die Sensoreinheit 20 weist zumindest einen Sensor 150 auf. Der Sensor 150 ist zur Sensierung der Kontaminationsrisikokenngröße vorgesehen. Der Sensor 150 ist in dem Anschlusskopf 22 angeordnet. Der Sensor 150 ist dem Anschluss 132 zugeordnet. Der Sensor 150 ist im Bereich des Anschlusses 132 angeordnet. Der Sensor 150 ist im vorliegenden Fall als ein Temperatursensor 24 ausgebildet.

Die Sensoreinheit 20 weist zumindest einen weiteren Sensor 152 auf. Der weitere Sensor 152 ist zur Sensierung der weiteren Kontaminationsrisikokenngröße vorgesehen. Der weitere Sensor 152 ist in dem Anschlusskopf 22 angeordnet. Der weitere Sensor 152 ist dem Anschluss 132 zugeordnet. Der weitere Sensor 152 ist im Bereich des Anschlusses 132 angeordnet. Der weitere Sensor 152 ist im vorliegenden Fall als ein Durchflusssensor 26 ausgebildet.

Die Entnahmeeinheit 18 weist zumindest eine Ventileinheit 140 auf. Die Ventileinheit 140 ist zu einer gezielten Trinkwasserentnahme vorgesehen. Die Ventileinheit 140 ist im vorliegenden Fall zumindest teilweise in den Anschlusskopf 22 integriert. Ferner ist die Ventileinheit 140 zumindest teilweise an dem Anschlusskopf 22 angeordnet, insbesondere an dem weiteren Anschluss 136.

Die Ventileinheit 140 umfasst zumindest ein Ventil 142. Im vorliegenden Fall ist das Ventil 142 als ein Schaltventil ausgebildet. Das Ventil 142 ist durch Schalten zu einer gezielten Probenentnahme öffenbar und/oder verschließbar. Zudem ist das Ventil 142 als ein Dosierventil ausgebildet. Das Ventil 142 ist zur Einstellung einer Probenmenge einer Trinkwasserprobe vorgesehen. Das Ventil 142 ist an dem Anschlusskopf 22 angeordnet, insbesondere an dem weiteren Anschluss 136.

Die Ventileinheit 140 weist zumindest ein weiteres Ventil 144 auf. Das weitere Ventil 144 ist als ein Rückschlagventil ausgebildet. Das weitere Ventil 144 ist in den Anschlusskopf 22 integriert. Das weitere Ventil 144 ist dem weiteren Anschluss 136 zugeordnet. Die Ventileinheit 140 könnte eine von der hier dargestellten abweichende Anzahl an Ventilen 142, 144 umfassen. Denkbar wäre, dass die Ventileinheit 140 nur ein Ventil 142, 144 umfasst, welches die Funktionen eines Dosierventils, eines Rückschlagventils und/oder eines Schaltventils vereint. Ferner könnte die Ventileinheit 140 vollständig in den Anschlusskopf 22 integriert sein.

Die Entnahmeeinheit 18 weist zumindest eine Entnahmeleitung 138 auf. Die Entnahmeleitung 138 verbindet die Trinkwasserleitung 12 mit einer Messvorrichtung 14 der Analyseeinrichtung 42 (vgl. Fig. 2). Die Entnahmeleitung 138 ist mit dem weiteren Anschluss 136 fluidtechnisch verbunden. Die Ventileinheit 140 ist zwischen dem Anschlusskopf 22 und der Entnahmeleitung 138 angeordnet. Die Entnahmeleitung 138 ist als ein Schlauch ausgebildet. Ferner ist die Entnahmeleitung 138 zumindest teilweise flexibel. Die Entnahmeleitung 138 ist zumindest teilweise aus einem flexiblen Material ausgebildet, wie beispielsweise einem Kunststoff, insbesondere Gummi. Denkbar ist, dass die Entnahmeeinheit 18 zumindest teilweise einstückig mit dem Anschlusskopf 22 und/oder der Ventileinheit 140 ausgebildet sein könnte. Beispielsweise könnte die Ventileinheit 140 in die Entnahmeleitung 138 integriert sein. Darunter, dass ein erstes Objekt und ein zweites Objekt "zumindest teilweise einstückig" ausgebildet sind, soll insbesondere verstanden werden, dass zumindest ein Element und/oder Teil des ersten Objekts und zumindest ein Element und/oder Teil des zweiten Objekts einstückig ausgebildet sind. Unter "einstückig" soll insbesondere zumindest stoffschlüssig verbunden verstanden werden, beispielsweise durch einen Schweißprozess, einen Klebeprozess, einen Anspritzprozess und/oder einen anderen, dem Fachmann als sinnvoll erscheinenden Prozess, und/oder vorteilhaft in einem Stück geformt verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling.

Die Probenentnahmevorrichtung 10 umfasst zumindest eine Filtereinheit 36. Die Filtereinheit 36 ist zu einer Makrofiltration des Trinkwassers 30 vorgesehen. Unter einer "Makrofiltration" soll insbesondere eine Filtration mit einer Porengröße von höchstens 1000 µm, vorzugsweise von höchstens 100 µm und besonders bevorzugt von höchstens 10 µm verstanden werden. Die Filtereinheit 36 ist zumindest teilweise in der Entnahmeeinheit 18 angeordnet. Die Filtereinheit 36 ist fluidtechnisch vor der Entnahmeleitung 138 angeordnet. Ferner ist die Filtereinheit 36 fluidtechnisch vor der Ventileinheit 140 angeordnet. Die Filtereinheit 36 umfasst zumindest ein Filterelement 146. Das Filterelement 146 ist austauschbar. Bei dem Filterelement 146 kann es sich beispielsweise um ein, insbesondere perlatorartiges, Filtersieb handeln. Das Filterelement 146 ist in dem Anschlusskopf 22 angeordnet. Das Filterelement 146 ist im Bereich des Anschlusses 136 angeordnet. Das Filterelement 146 weist eine Porengröße von höchstens 1000 µm auf. Ferner kann die Filtereinheit 36 zwei oder mehrere insbesondere verschieden voneinander ausgebildete Filterelemente 146 umfassen. Beispielsweise könnte die Filtereinheit 36 Filterelemente 146 verschiedener Porengrößen umfassen. Die Filtereinheit 36 könnte auch als Teil der Ventileinheit 140 angeordnet sein oder zwischen Anschlusskopf 22, Ventileinheit 140 und/oder Entnahmeleitung 138 angeordnet sein.

Die Probenentnahmevorrichtung 10 umfasst zumindest eine Desinfektionseinheit 38. Die Desinfektionseinheit 38 ist zu einer Beseitigung bzw. Abtötung von Mikroorganismen 16 vorgesehen. Die Desinfektionseinheit 38 ist zu einer Desinfektion zumindest der Entnahmeeinheit 18 vorgesehen. Mittels der Desinfektionseinheit 38 kann zwischen Messungen einer Trinkwasserkontamination die Entnahmeeinheit 18 desinfiziert werden, um eine Verfälschung einer Messung durch sich in der Entnahmeeinheit 18 anlagernde und/oder vermehrende Mikroorganismen 16 zu vermeiden.

Im vorliegenden Fall ist die Desinfektionseinheit 38 zu einer physikalischen Desinfektion vorgesehen. Die Desinfektionseinheit 38 ist zu einer Erwärmung der Entnahmeeinheit 18 auf zumindest 70°C vorgesehen. Die Desinfektionseinheit 38 umfasst zumindest ein Temperierelement 40. Die Entnahmeleitung 138 bildet die Desinfektionseinheit 38 aus. Die Entnahmeleitung 138 ist beheizbar, insbesondere als ein Temperierelement 40, ausgebildet. Alternativ oder zusätzlich ist denkbar, dass die Desinfektionseinheit 38 ein separat von der Entnahmeleitung 138 ausgebildetes Desinfektionselement aufweist. Beispielsweise könnte es sich bei dem Desinfektionselement um eine Heizspule handeln. Ferner ist denkbar, dass das Desinfektionselement als ein Peltierelement ausgebildet sein kann.

Alternativ oder zusätzlich könnte die Desinfektionseinheit 38 zu einer physikalischen Desinfektion durch Bestrahlung mit elektromagnetischer Strahlung, wie beispielsweise UV-Licht, vorgesehen sein. Für diesen Fall könnte das Desinfektionselement als eine UV-Lichtquelle ausgebildet sein. Vorzugsweise wäre für diesen Fall die Entnahmeleitung 138 zumindest für UV-Licht durchlässig ausgebildet. Denkbar ist, dass die Desinfektionseinheit 38 zu einer chemischen Desinfektion vorgesehen sein könnte. Unter einer "chemischen Desinfektion" soll insbesondere eine Desinfektion mittels eines Oxidationsmittels, wie beispielsweise Chlor, Chloroxid, Wasserstoffperoxid, Dimethyldicarbonat, Silberionen und/oder Ozon, verstanden werden. Insbesondere könnte ein zu einer chemischen Desinfektion und/oder zu einer Desinfektion mit UV-Licht vorgesehenes Desinfektionselement zwischen dem Anschlusskopf 22 und der Entnahmeleitung 138 angeordnet sein.

Die Probenentnahmevorrichtung 10 weist zumindest eine Dispergiereinheit 28 auf. Die Dispergiereinheit 28 ist dazu vorgesehen, Mikroorganismen 16 im Trinkwasser 30 zu dispergieren. Die Dispergiereinheit 28 ist dazu vorgesehen, Agglomerate und/oder Aggregate von Mikroorganismen 16, welche insbesondere bei einer Ausbildung eines Biofilms auftreten, zu vorzugsweise einzelnen voneinander getrennten Mikroorganismen 16 zu zerlegen. Die Dispergiereinheit 28 ist im vorliegenden Fall als ein Ultraschalldispergierer ausgebildet. Die Dispergiereinheit 28 dispergiert durch Einleiten von Ultraschall in die Entnahmeeinheit 18 Mikroorganismen 16 im Trinkwasser 30. Dazu stellt die Dispergiereinheit 28 insbesondere Ultraschall mit einer Frequenz von zumindest 16 kHz, bevorzugt von zumindest 18 kHz, besonders bevorzugt von zumindest 20 kHz und ganz besonders bevorzugt von zumindest 30 kHz bereit. Die Dispergiereinheit 28 ist an der Entnahmeeinheit 18 angeordnet. Die Dispergiereinheit 28 ist an einem Ende der Entnahmeleitung 138 angeschlossen.

Die Probenentnahmevorrichtung 10 weist zumindest eine weitere Sensoreinheit 156 auf. Die weitere Sensoreinheit 156 ist dazu vorgesehen, einen Durchfluss zur Dosierung einer Trinkwasserprobe durch die Entnahmeeinheit 18, insbesondere die Entnahmeleitung 138, zu sensieren. Die weitere Sensoreinheit 156 ist als Ultraschall-Durchflussmesser ausgebildet.

Die Probenentnahmevorrichtung 10 umfasst zumindest eine Entkopplungseinheit 32. Die Entkopplungseinheit 32 ist zu einer Schwingungsentkopplung der sich entlang der Entnahmeeinheit 18 mechanisch ausbreitenden Schwingungen vorgesehen. Die Entkopplungseinheit 32 ist dazu vorgesehen, die Dispergiereinheit 28 schwingungsmechanisch zu entkoppeln. Die Entkopplungseinheit 32 ist dazu vorgesehen, die Sensoreinheit 20 zumindest teilweise schwingungsmechanisch zu entkoppeln. Die Entkopplungseinheit 32 ist dazu vorgesehen, die Dispergiereinheit 28 und die Sensoreinheit 20 zumindest teilweise schwingungsmechanisch voneinander zu entkoppeln. Die Entkopplungseinheit 32 ist dazu vorgesehen, die Sensoreinheit 20 thermisch von übrigen Komponenten der Probenentnahmevorrichtung 10, insbesondere von der Dispergiereinheit 28, zu entkoppeln. Zur Entkopplung weist die Entkopplungseinheit 32 zumindest ein Entkopplungselement 154 auf.

Im vorliegenden Fall sind die Entkopplungseinheit 32 und die Entnahmeeinheit 18 zumindest teilweise einstückig ausgebildet. Im vorliegenden Fall ist die Entkopplungseinheit 32 zumindest teilweise von der Entnahmeleitung 138 ausgebildet. Die Entnahmeleitung 138 bildet das Entkopplungselement 154 aus.

Die Entnahmeeinheit 18 der Probenentnahmevorrichtung 10 ist fluidtechnisch mit einem Trinkwasserprobenanschluss 214 der Messvorrichtung 14 verbunden. Der Trinkwasserprobenanschluss 214 ist beabstandet mit einem Trinkwasserprobenauslass 216 der Messvorrichtung 14 verbunden.

Die Analyseeinrichtung 42 weist zumindest eine Steuereinheit 34 auf. Die Steuereinheit 34 ist Teil der Probenentnahmevorrichtung 10. Die Steuereinheit 34 ist zur Steuerung weiterer Komponenten der Analyseeinrichtung 42, insbesondere der Probenentnahmevorrichtung 10, der Messvorrichtung 14 und/oder der Kartuschenvorrichtung 44, vorgesehen. Ferner steht die Steuereinheit 34 in kommunizierender Verbindung mit der externen Einheit 128. Die Steuereinheit 34 steht in einer kabellosen kommunizierenden Verbindung mit der externen Einheit 128, beispielsweise über Funk, WLAN, Bluetooth, GSM o. dgl. Die Steuereinheit 34 stellt der externen Einheit 128 Daten zur Verfügung. Die Steuereinheit 34 ist durch die externe Einheit 128 ansteuerbar. Im vorliegenden Fall ist die Steuereinheit 34 Teil der Messvorrichtung 14 (vgl. Fig. 2).

Die Steuereinheit 34 umfasst zumindest eine Prozessoreinheit (nicht dargestellt). Ferner umfasst die Steuereinheit 34 zumindest eine Speichereinheit (nicht dargestellt). Die Prozessoreinheit und die Speichereinheit sind der Übersichtlichkeit halber in den Figuren nicht dargestellt. Die Steuereinheit 34 umfasst ferner zumindest ein Betriebsprogramm. Das Betriebsprogramm ist in der Prozessoreinheit ausführbar. Ferner ist das Betriebsprogramm auf der Speichereinheit hinterlegt. Das Betriebsprogramm ist zur Ausführung eines Verfahrens zum Betrieb der Analyseeinrichtung 42, insbesondere der Probenentnahmevorrichtung 10, der Messvorrichtung 14 und/oder der Kartuschenvorrichtung 44, vorgesehen.

Die Steuereinheit 34 ist dazu vorgesehen, zumindest anhand der Kontaminationsrisikokenngröße eine Trinkwasserkontamination zu prognostizieren. Im vorliegenden Fall ist die Steuereinheit 34 dazu vorgesehen, zusätzlich anhand der weiteren Kontaminationsrisikokenngröße die Trinkwasserkontamination zu prognostizieren. Vorzugsweise nutzt die Steuereinheit 34 ein Kontaminationskennfeld 158. Das Kontaminationskennfeld 158 ist in der Steuereinheit 34, insbesondere der Speichereinheit der Steuereinheit 34, hinterlegt. Bei dem Kontaminationskennfeld 158 handelt es sich um ein Kennfeld, in welchem zumindest Werte zumindest der Kontaminationsrisikokenngrößen und der weiteren Kontaminationsrisikokenngröße gegeneinander aufgetragen sind. Ferner sind die Kontaminationsrisikokenngrößen in dem Kontaminationskennfeld 158 einer Wahrscheinlichkeit einer Trinkwasserkontamination zugeordnet. Ferner ist denkbar, dass die Steuereinheit 34 eine Wahrscheinlichkeit einer Trinkwasserkontamination anhand einer Kontaminationskennlinie bestimmt, welche zumindest Werte einer Kontaminationsrisikokenngröße umfasst, welche einer jeweiligen Wahrscheinlichkeit einer Trinkwasserkontamination zugeordnet sind.

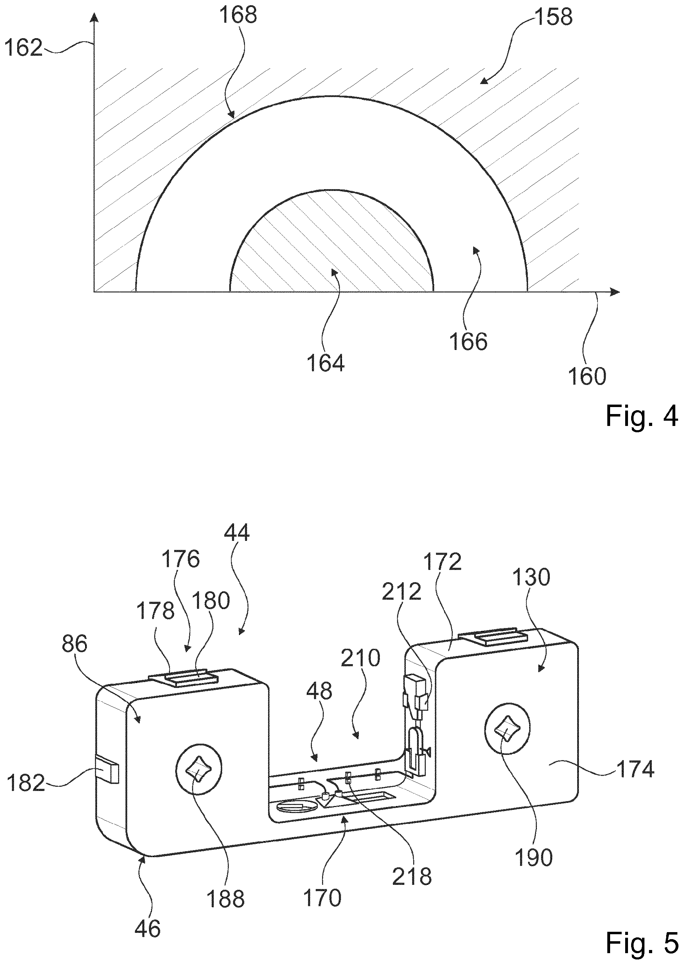

Fig. 4 zeigt ein Diagramm eines beispielhaften Kontaminationskennfelds 158. Das Diagramm weist eine Abszissenachse 160 auf. An der Abszissenachse 160 ist die Kontaminationsrisikokenngröße aufgetragen. Ferner weist das Diagramm eine Ordinatenachse 162 auf. An der Ordinatenachse 162 ist die weitere Kontaminationsrisikokenngröße aufgetragen. Im vorliegenden Fall sind die Kontaminationsrisikokenngröße und die weitere Kontaminationsrisikokenngröße durch in der Steuereinheit 34 hinterlegte Vergleichswerte mit einer jeweiligen Wahrscheinlichkeit einer Trinkwasserkontamination verknüpft. Ferner könnten diese durch eine mathematische Gleichung miteinander verknüpft sein, wie beispielsweise über eine Kreis- und/oder Ellipsengleichung. Das Kontaminationskennfeld 158 weist zumindest verschiedene Wahrscheinlichkeitsbereiche 164, 166, 168 einer Trinkwasserkontamination auf. Die in Fig. 4 dargestellten verschiedenen Wahrscheinlichkeitsbereiche 164, 166, 168, insbesondere Wahrscheinlichkeiten und/oder Wertebereiche der Wahrscheinlichkeitsbereiche 164, 166, 168, sind insbesondere lediglich beispielhaft zu verstehen. Insbesondere können die verschiedenen Wahrscheinlichkeitsbereiche 164, 166, 168 in einer von der in Fig. 4 dargestellten abweichenden Ausführungsform von den angegebenen Wahrscheinlichkeiten und/oder Wertebereichen abweichende Wahrscheinlichkeiten und/oder Wertebereiche aufweisen.

Das Kontaminationskennfeld 158 weist einen Wahrscheinlichkeitsbereich 164 auf. In dem Wahrscheinlichkeitsbereich 164 wird eine Trinkwasserkontamination mit einer beispielhaften Wahrscheinlichkeit von zumindest 85 % prognostiziert. Der Wahrscheinlichkeitsbereich 164 tritt im vorliegenden Fall bei einem beispielhaften Wertebereich der Kontaminationsrisikokenngröße von wenigstens 27° C und maximal 60°C auf. Der Wahrscheinlichkeitsbereich 164 tritt im vorliegenden Fall bei einem beispielhaften Wertebereich der weiteren Kontaminationsrisikokenngröße von wenigstens 0 % und maximal 45 % eines maximalen Durchflusses von Trinkwasser 30 durch die Trinkwasserleitung 12 auf.

Das Kontaminationskennfeld 158 weist einen weiteren Wahrscheinlichkeitsbereich 166 auf. In dem weiteren Wahrscheinlichkeitsbereich 166 wird eine Trinkwasserkontamination mit einer beispielhaften Wahrscheinlichkeit von zumindest 50 % prognostiziert. Die Wahrscheinlichkeit einer Trinkwasserkontamination des weiteren Wahrscheinlichkeitsbereichs 166 ist geringer als die Wahrscheinlichkeit einer Trinkwasserkontamination des Wahrscheinlichkeitsbereichs 164. Der weitere Wahrscheinlichkeitsbereich 166 tritt im vorliegenden Fall bei einem beispielhaften Wert der Kontaminationsrisikokenngröße von wenigstens 20° C und maximal 65° C, insbesondere abzüglich des Wertebereichs des Wahrscheinlichkeitsbereichs 164, auf. Der weitere Wahrscheinlichkeitsbereich 166 tritt im vorliegenden Fall bei einem beispielhaften Wertebereich der weiteren Kontaminationsrisikokenngröße von wenigstens 0 % und maximal 66 % eines maximalen Durchflusses von Trinkwasser 30 durch die Trinkwasserleitung 12, insbesondere abzüglich des Wertebereichs des Wahrscheinlichkeitsbereichs 164 auf.

Das Kontaminationskennfeld 158 weist einen zusätzlichen Wahrscheinlichkeitsbereich 168 einer Trinkwasserkontamination auf. In dem zusätzlichen Wahrscheinlichkeitsbereich 168 wird eine Trinkwasserkontamination mit einer beispielhaften Wahrscheinlichkeit von zumindest 30 % prognostiziert. Die Wahrscheinlichkeit einer Trinkwasserkontamination des zusätzlichen Wahrscheinlichkeitsbereichs 168 ist geringer als die Wahrscheinlichkeit einer Trinkwasserkontamination des weiteren Wahrscheinlichkeitsbereichs 166. Der zusätzliche Wahrscheinlichkeitsbereich 168 tritt im vorliegenden Fall bei einem beispielhaften Wert der Kontaminationsrisikokenngröße von zumindest 0° C und höchstens 100° C, insbesondere geschnitten mit den Wertebereichen des weiteren Wahrscheinlichkeitsbereichs 166 auf. Der zusätzliche Wahrscheinlichkeitsbereich 168 tritt im vorliegenden Fall bei einem beispielhaften Wertebereich der weiteren Kontaminationsrisikokenngröße von wenigstens 0 % und maximal 100 % eines maximalen Durchflusses von Trinkwasser 30 durch die Trinkwasserleitung 12, insbesondere geschnitten mit den Wertebereichen des weiteren Wahrscheinlichkeitsbereichs 166 auf.

Im vorliegenden Fall sind die Wahrscheinlichkeiten einer Kontamination der jeweiligen Wahrscheinlichkeitsbereiche 164, 166, 168 diskretisiert. Es wird zwischen drei verschiedenen diskreten Wahrscheinlichkeiten unterschieden. Alternativ ist denkbar, dass eine Wahrscheinlichkeit kontinuierlich anhand der Werte der Kontaminationsrisikokenngrößen bestimmbar ist.

Die Steuereinheit 34 ist dazu vorgesehen, eine Probenentnahme abhängig von zumindest einer prognostizierten Trinkwasserkontamination durchzuführen. Im vorliegenden Fall führt die Steuereinheit 34 die Probenentnahme abhängig von einer anhand der Kontaminationsrisikokenngrößen bestimmten Wahrscheinlichkeit einer Trinkwasserkontamination der jeweiligen Wahrscheinlichkeitsbereiche 164, 166, 168 durch. Die Steuereinheit 34 führt eine Häufigkeit einer Probenentnahme abhängig von der prognostizierten Trinkwasserkontamination, insbesondere einer Wahrscheinlichkeit der Trinkwasserkontamination, durch. Dabei ist eine jeweilige Häufigkeit einer Probenentnahme abhängig von der Wahrscheinlichkeit der Trinkwasserkontamination vorteilhaft unter Gewichtung mit einem Faktor einer maximalen Probenentnahmehäufigkeit. Beispielsweise führt die Steuereinheit 34 bei einer Wahrscheinlichkeit der Trinkwasserkontamination des Wahrscheinlichkeitsbereichs 164 häufiger einer Probenentnahme durch als bei einer Wahrscheinlichkeit der Trinkwasserkontamination des Wahrscheinlichkeitsbereichs 166. Ferner führt die Steuereinheit 34 bei einer Wahrscheinlichkeit der Trinkwasserkontamination des weiteren Wahrscheinlichkeitsbereichs 166 häufiger eine Probenentnahme durch als bei einer Trinkwasserkontaminationswahrscheinlichkeit des zusätzlichen Wahrscheinlichkeitsbereichs 168. Eine Häufigkeit einer Probenentnahme bei einer Wahrscheinlichkeit der Trinkwasserkontamination des zusätzlichen Wahrscheinlichkeitsbereichs 168 entspricht dabei einer Häufigkeit von zumindest einmal in drei Jahren, insbesondere gemäß der Trinkwasserverordnung TrinkwV 2001. Alternativ oder zusätzlich ist denkbar, dass die Steuereinheit 34 eine Probenentnahme dann automatisiert durchführen kann, wenn die Wahrscheinlichkeit der Trinkwasserkontamination einen hinterlegten Referenzwert überschreitet.

Fig. 5 zeigt einen Teil der Kartuschenvorrichtung 44 in einer schematischen perspektivischen Darstellung. Die Kartuschenvorrichtung 44 ist austauschbar. Die Kartuschenvorrichtung 44 ist für die Messvorrichtung 14 zur Messung einer Trinkwasserkontamination in der Trinkwasserleitung 12 durch Mikroorganismen 16 vorgesehen. Die Kartuschenvorrichtung 44 weist zumindest ein Kartuschengehäuse 46 auf. Das Kartuschengehäuse 46 ist kassettenartig ausgebildet. Das Kartuschengehäuse 46 weist eine brillenartige Form auf. Das Kartuschengehäuse 46 weist zumindest einen Lagerabschnitt 86 auf. Ferner weist das Kartuschengehäuse 46 einen weiteren Lagerabschnitt 130 auf. Der Lagerabschnitt 86 und der weitere Lagerabschnitt 130 sind zumindest im Wesentlichen spiegelsymmetrisch zueinander. Das Kartuschengehäuse 46 weist einen Steg 170 auf. Der Steg 170 verbindet den Lagerabschnitt 86 und den weiteren Lagerabschnitt 130 miteinander.

Das Kartuschengehäuse 46 weist einen Behälter 172 auf. Der Behälter 172 ist zur Anordnung weiterer Komponenten der Kartuschenvorrichtung 44 vorgesehen. Ferner weist das Kartuschengehäuse 46 einen Deckel 174 auf. Der Deckel 174 verschließt den Behälter 172.

Das Kartuschengehäuse 46 ist mit der Messvorrichtung 14 koppelbar (vgl. Fig. 4). Zur Kopplung weist die Kartuschenvorrichtung 44 zumindest eine Koppeleinheit 176 auf. Im vorliegenden Fall ist die Koppeleinheit 176 als eine Schnellkopplung ausgebildet. Die Koppeleinheit 176 weist zumindest ein Rastelement 178 auf. Das Rastelement 178 ist an dem Kartuschengehäuse 46, insbesondere dem Behälter 172, angeordnet. Im vorliegenden Fall ist das Rastelement 178 einstückig mit dem Kartuschengehäuse 46, insbesondere dem Behälter 172, ausgebildet. Das Rastelement 178 ist im Bereich des Lagerabschnitts 86 angeordnet. Das Rastelement 178 ist dazu vorgesehen, mit einem korrespondierenden Rastelement (nicht dargestellt) der Messvorrichtung 14 eine kraft- und/oder formschlüssige Verbindung einzugehen. Ferner weist die Koppeleinheit 176 ein weiteres Rastelement auf. Das weitere Rastelement ist zumindest im Wesentlichen identisch zu dem Rastelement 178 ausgebildet. Das weitere Rastelement ist im Bereich des weiteren Lagerabschnitts 130 angeordnet.

Ferner weist die Koppeleinheit 176 zumindest ein Betätigungselement 180 auf. Das Betätigungselement 180 ist zu einem Aufheben einer Kopplung des Kartuschengehäuses 46 mit der Messvorrichtung 14 vorgesehen. Im vorliegenden Fall ist das Betätigungselement 180 als ein Hebel ausgebildet. Das Betätigungselement 180 ist einstückig mit dem Rastelement 178 verbunden. Ferner weist die Koppeleinheit 176 ein weiteres Betätigungselement auf. Das weitere Betätigungselement ist zumindest im Wesentlichen identisch zu dem Betätigungselement ausgebildet. Das weitere Betätigungselement ist im Bereich des weiteren Lagerabschnitts 130 angeordnet.

Zudem weist die Koppeleinheit 176 zumindest ein Führungselement 182 auf. Das Führungselement 182 ist zu einer Führung des Kartuschengehäuses 46 bei einer Kopplung mit der Messvorrichtung 14 vorgesehen. Das Führungselement 182 ist an dem Kartuschengehäuse 46, insbesondere dem Behälter 172, angeordnet. Im vorliegenden Fall ist das Führungselement 182 einstückig mit dem Kartuschengehäuse 46, insbesondere dem Behälter 172, ausgebildet. Das Führungselement 182 ist im Bereich des Lagerabschnitts 86 angeordnet. Ferner weist die Koppeleinheit 176 ein weiteres Führungselement auf. Das weitere Führungselement ist zumindest im Wesentlichen identisch zu dem Führungselement 182 ausgebildet. Das weitere Führungselement ist im Bereich des weiteren Lagerabschnitts 130 angeordnet. Alternativ oder zusätzlich zu der in Fig. 5 beispielhaft dargestellten Ausbildungsform ist denkbar, dass die Koppeleinheit 176 zumindest eine Schraube, zumindest eine Schweißverbindung o. dgl. zu einer Kopplung des Kartuschengehäuses 46 mit der Messvorrichtung 14 aufweist.

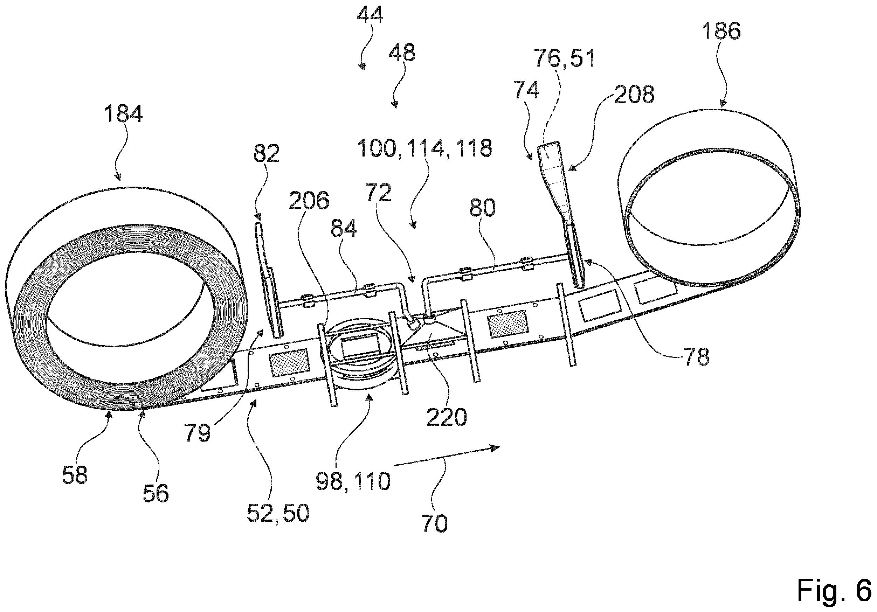

Ferner weist die Kartuschenvorrichtung 44 zumindest eine Versuchseinheit 48 auf. Die Versuchseinheit 48 ist zumindest teilweise in dem Kartuschengehäuse 46 angeordnet. Die Versuchseinheit 48 weist zumindest ein Verbrauchsmaterial 50 auf. Das Verbrauchsmaterial 50 ist zur Durchführung zumindest eines Versuchs zur Messung der Trinkwasserkontamination vorgesehen. Ferner weist die Versuchseinheit 48 zumindest ein weiteres Verbrauchsmaterial 51 auf (vgl. Fig. 6).

Fig. 6 zeigt einen Teil der Kartuschenvorrichtung 44 ohne das Kartuschengehäuse 46 in einer schematischen perspektivischen Darstellung. Die Versuchseinheit 48 weist zumindest ein Versuchsband 52 auf. Zumindest bei dem Versuchsband 52 handelt es sich um ein Verbrauchsmaterial 50 der Versuchseinheit 48.

In zumindest einem Lagerzustand des Versuchsbands 52 ist das Versuchsband 52 zumindest abschnittsweise aufgerollt gelagert. Vorzugsweise weist die Versuchseinheit 48 zumindest eine Abrollspule 184 auf. Die Abrollspule 184 ist im Lagerabschnitt 86 angeordnet (vgl. Fig. 5). Im Lagerzustand ist das Versuchsband 52 zumindest teilweise auf die Abrollspule 184 aufgerollt. Zur Durchführung eines Versuchs zur Messung einer Trinkwasserkontamination kann das Versuchsband 52 von der Abrollspule 184 abgerollt werden. Durch die aufgerollte Lagerung ist in dem Lagerzustand des Versuchsbands 52 zumindest ein Versuchsbandabschnitt 56 des Versuchsbands 52 zumindest teilweise von zumindest einem weiteren Versuchsbandabschnitt 58 des Versuchsbands 52 bedeckt.

Ferner weist die Versuchseinheit 48 zumindest eine Aufrollspule 186 auf. Die Aufrollspule 186 ist im weiteren Lagerabschnitt 130 angeordnet. Im Lagerzustand ist das Versuchsband 52 zumindest teilweise auf die Aufrollspule 186 aufgerollt. Nach Durchführung eines Versuchs zur Messung einer Trinkwasserkontamination kann das Versuchsband 52 auf die Aufrollspule 186 aufgerollt werden. Durch die aufgerollte Lagerung ist in dem Lagerzustand des Versuchsbands 52 zumindest ein Versuchsbandabschnitt 56 des Versuchsbands 52 zumindest teilweise von zumindest einem weiteren Versuchsbandabschnitt 58 des Versuchsbands 52 bedeckt.

Durch ein gleichzeitiges Abrollen und Aufrollen des Versuchsbands 52 wird dieses entlang seiner Haupterstreckungsrichtung 70 im abgerollten Zustand bewegt.

Um das Versuchsband 52 aufzurollen und/oder abzurollen, weisen die Aufrollspule 186 und die Abrollspule 184 jeweils eine Achsaufnahme 188, 190 auf. Die Achsaufnahmen 188, 190 sind rotationssymmetrisch. Die Achsaufnahmen 188, 190 weisen jeweils einen kreuzförmigen Querschnitt auf. Die Achsaufnahmen 188, 190 sind jeweils zur Aufnahme einer Antriebsachse 192, 194 vorgesehen. Die Messvorrichtung 14 umfasst dabei zwei korrespondierend ausgebildete Antriebsachsen 192, 194 (vgl. Fig. 2).

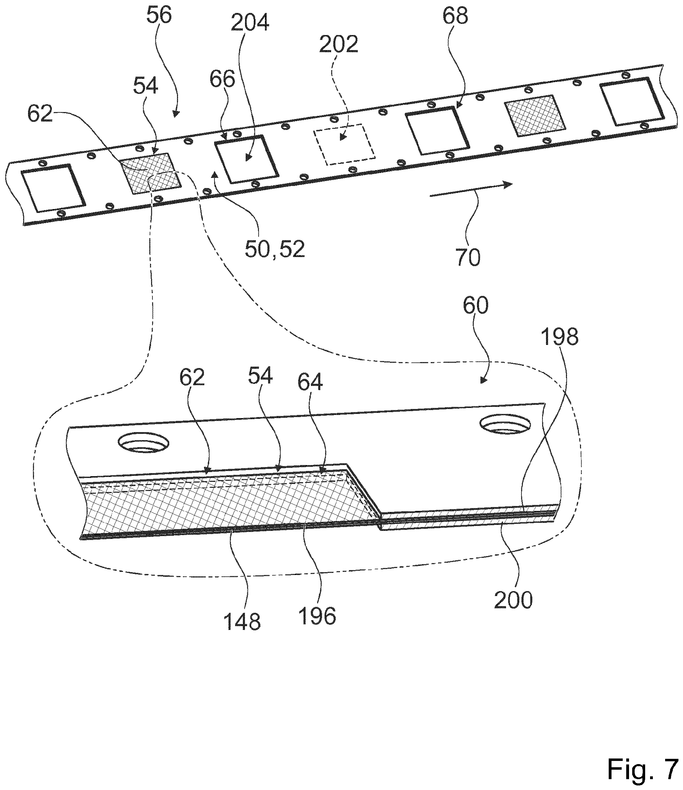

Fig. 7 zeigt einen Teil des Versuchsbands 52 im abgerollten Zustand des Versuchsbandabschnitts 56 in einer schematischen perspektivischen Darstellung.

Das Versuchsband 52 weist einen Schichtaufbau 60 auf. Das Versuchsband 52 weist zumindest eine Filterschicht 196 auf. Die Filterschicht 196 ist zu einer Filtrierung einer Trinkwasserprobe bei einer Messung vorgesehen. Die Filterschicht 196 weist einen Porendurchmesser von höchstens 0,45 µm auf. Alternativ ist denkbar, dass die Filterschicht 196 einen Porendurchmesser von höchstens 0,2 µm aufweist. Die Filterschicht 196 weist insbesondere eine Schichtdicke von höchstens 0,1 mm auf. Die Filterschicht 196 ist von einem Filtervlies ausgebildet. Die Filterschicht 196 besteht zumindest teilweise aus einem hygroskopischen Material. Die Filterschicht 196 ist frei von einem hydrophilen Material.

Ferner weist das Versuchsband 52 zumindest eine Trägerschicht 198 auf. Die Trägerschicht 198 ist dazu vorgesehen, zumindest die Filterschicht 196 zu tragen. Die Trägerschicht 198 weist eine Schichtdicke von höchstens 0,5 mm auf. Die Trägerschicht 198 besteht zumindest teilweise aus einem Kunststoff, wie beispielsweise Polyethylen und/oder Polypropylen. Die Trägerschicht 198 weist eine glatte Oberfläche auf. Die Trägerschicht 198 ist mit der Filterschicht 196 stoffschlüssig verbunden. Im vorliegenden Fall ist die Trägerschicht 198 mit der Filterschicht 196 verschweißt. Die Trägerschicht 198 ist mit der Filterschicht 196 durch einen Kalandrierprozess verschweißt. Alternativ könnten diese auch miteinander verklebt sein.

Ferner weist das Versuchsband 52 zumindest eine weitere Trägerschicht 200 auf. Die weitere Trägerschicht 200 ist zumindest im Wesentlichen identisch zur Trägerschicht 198 ausgebildet. Alternativ könnte die weitere Trägerschicht 200 jedoch auch von der Trägerschicht 198 verschieden ausgebildet sein. Die Filterschicht 196 ist zwischen den Trägerschichten 198, 200 eingeschlossen. Demnach liegt im vorliegenden Fall der Schichtaufbau 60 in Form einer Sandwichbauweise vor. Die Filterschicht 196 ist mit der Trägerschicht 198 und mit einer Stützschicht 148 des Versuchsbands 52 verschweißt. Die Stützschicht 148 ist zwischen den Trägerschichten 198, 200 angeordnet. Die Stützschicht 148 ist dazu vorgesehen, die Filterschicht 196 zu stabilisieren. Die Stützschicht 148 ist als ein, insbesondere grobmaschiges, Gewebe ausgebildet. Die Stützschicht 148 weist eine Schichtdicke zumindest im Wesentlichen analog zu der Schichtdicke der Filterschicht 196 auf.

Das Versuchsband 52 umfasst zumindest ein Versuchsfeld 54. Das Versuchsfeld 54 umfasst zumindest einen Filter 62. Der Filter 62 ist zur Abscheidung von Mikroorganismen 16 aus Trinkwasser 30 vorgesehen. Im vorliegenden Fall ist der Filter 62 von der Filterschicht 196 des Versuchsbands 52 ausgebildet.

Das Versuchsfeld 54 weist eine Senke 64 auf. Die Senke 64 ist zur Aufnahme von Mikroorganismen 16 aus der Trinkwasserprobe vorgesehen. Die Senke 64 ist durch bereichsweises Entfernen, insbesondere Ausstanzen, der Trägerschicht 198, 200 des Versuchsbands 52 im Bereich des Versuchsfelds 54 gebildet. Die Senke 64 legt die an der Trägerschicht 198 angeordnete Filterschicht 196 frei. Derart bildet die Filterschicht 196 im Bereich des Versuchsfelds 54 den Filter 62 des Versuchsfelds 54 aus. Durch Durchspülen des Filters 62 mit der Trinkwasserprobe werden insbesondere Mikroorganismen 16 an dem Filter 62 zurückgehalten, welche sich in der Senke 64 ansammeln.

Ferner weist das Versuchsband 52 zumindest ein Reinigungsfeld 66 auf. Das Reinigungsfeld 66 ist zur Spülung mit Trinkwasser 30 vorgesehen. Das Reinigungsfeld 66 weist eine größere Grundfläche auf als das Versuchsfeld 54. Das Reinigungsfeld 66 weist quer zu einer Haupterstreckungsrichtung 70 des Versuchsbands 52 eine größere Erstreckung auf als das Versuchsfeld 54. Ferner wird vorgeschlagen, dass das Reinigungsfeld 66 zumindest eine Ausnehmung 204 aufweist. Bei der Ausnehmung 204 handelt es sich um eine volle Ausnehmung. Die Ausnehmung 204 betrifft alle Schichten, aus welchen sich das Versuchsband 52 zusammensetzt. Im Bereich des Reinigungsfelds 66 sind die Trägerschicht 198, die weitere Trägerschicht 200 und die Filterschicht 196 entfernt und insbesondere ausgestanzt. Im Bereich des Reinigungsfelds 66 ist die Filterschicht 196 entlang einer vollständigen Erstreckung des Versuchsbands 52 quer zu der Haupterstreckungsrichtung 70 des Versuchsbands 52 entfernt. Durch die Ausnehmung 204 des Reinigungsfelds 66 ist das Versuchsfeld 54 entlang des Versuchsbands 52 fluidtransporttechnisch, insbesondere über Kapillarkräfte, getrennt von weiteren Versuchsfeldern des Versuchsbands 52.

Das Versuchsfeld 54 und das Reinigungsfeld 66 sind entlang der Haupterstreckungsrichtung 70 des Versuchsbands 52 versetzt zueinander angeordnet. Ferner ist ein Trennfeld 202 zwischen dem Reinigungsfeld 66 und einem weiteren Reinigungsfeld 68 angeordnet. Das Versuchsfeld 54 und das Reinigungsfeld 66 sind in Haupterstreckungsrichtung 70 des Versuchsbands 52 alternierend zueinander angeordnet.

Ferner weist das Versuchsband 52 zumindest ein Trennfeld 202 auf. Das Trennfeld 202 ist dazu vorgesehen, das Reinigungsfeld 66 und das weitere Reinigungsfeld 68 voneinander zu beabstanden. Ferner ist das Trennfeld 202 dazu vorgesehen, im Lagerzustand das Versuchsfeld 54 zu bedecken. Das Trennfeld 202 ist in Fig. 7 durch eine unterbrochene Linie markiert. Das Trennfeld 202 weist einen vollen Schichtaufbau 60 des Versuchsbands 52 auf und ist frei von Senken oder Ausnehmungen ausgebildet.

Das Versuchsfeld 54, das Trennfeld 202 und das Reinigungsfeld 66 sind dabei in einer sich insbesondere wiederholenden Versuchsbandsequenz in Haupterstreckungsrichtung 70 des Versuchsbands 52 angeordnet. Das Versuchsband 52 umfasst mehrere aneinander angeordnete Versuchsbandsequenzen. Eine Versuchsbandsequenz umfasst ein Versuchsfeld 54, ein Reinigungsfeld 66, ein Trennfeld 202 und ein weiteres Reinigungsfeld 68.

Die Kartuschenvorrichtung 44 weist zumindest eine Dichteinheit 72 auf (vgl. Fig. 6). Die Dichteinheit 72 dichtet die Versuchseinheit 48 zumindest abschnittsweise fluiddicht ab. Die Dichteinheit 72 liegt dichtend an zumindest einem Versuchsbandabschnitt 56 des Versuchsbands 52 an. Die Dichteinheit 72 trennt verschiedene Versuchsstationen 98, 100, 102 der Analyseeinrichtung 42 voneinander fluiddicht ab. Unter einer "Versuchsstation" 98, 100, 102, soll insbesondere eine Station verstanden werden, an welcher zumindest ein Versuchsteil zur Durchführung des Versuchs vollzogen wird und entlang welcher vorzugsweise das Versuchsband 52 bei einem Versuch zur Messung bewegt wird. Die Dichteinheit 72 dichtet einen unbedeckten Versuchsbandabschnitt 56 des Versuchsbands 52 ab. Die Dichteinheit 72 weist zumindest eine Dichtung 206 auf.

Die Dichtung 206 ist als eine Dichtlippe ausgebildet. Die Dichtung 206 kontaktiert das Versuchsband 52 unmittelbar, wobei insbesondere lediglich die Senke 64 des Versuchsfelds 54 nicht in Kontakt mit der Dichtung 206 steht.

Ferner weist die Dichteinheit 72 zumindest eine weitere Dichtung auf. Die weitere Dichtung ist zumindest im Wesentlichen identisch mit der Dichtung 206 ausgebildet. Im vorliegenden Fall weist die Dichteinheit 72 insgesamt vier Dichtungen 206 auf. Jeweils zwei Dichtungen 206 dichten eine der Versuchsstationen 98, 100, 102 fluiddicht ab. Der Übersichtlichkeit halber ist in den Figuren nur eine Dichtung 206 mit einem Bezugszeichen versehen. Die Dichtung 206 und die weitere Dichtung sind insbesondere zueinander entlang der Haupterstreckungsrichtung 70 des Versuchsbands 52 versetzt angeordnet. Im vorliegenden Fall weist die Dichteinheit 72 vier Dichtungen 206 auf.

Die Versuchseinheit 48 umfasst zumindest eine Versorgungseinheit 74. Die Versorgungseinheit 74 ist zur Bereitstellung von Markern 76 zur Markierung der Mikroorganismen 16 vorgesehen. Bei den Markern 76 handelt es sich um das weitere Verbrauchsmaterial 51. Die Versorgungseinheit 74 ist zumindest teilweise an dem Kartuschengehäuse 46 angeordnet. Im vorliegenden Fall ist die Versorgungseinheit 74 an einer Außenseite des Kartuschengehäuses 46 angeordnet. Die Versorgungseinheit 74 ist im Bereich des weiteren Lagerabschnitts 130 angeordnet. Alternativ ist auch denkbar, dass die Versorgungseinheit 74 zumindest teilweise innerhalb des Kartuschengehäuses 46 angeordnet sein könnte.

Die Versorgungseinheit 74 weist insbesondere eine Speichereinheit 208 auf. In der Speichereinheit 208 sind die Marker 76 gespeichert. Die Marker 76 sind Verbrauchsmaterial 51, welches ausgetauscht oder nachgefüllt werden kann. Beispielsweise kann die Speichereinheit 208 durch eine neue mit neuen Markern gefüllte Speichereinheit ersetzbar sein. Ferner ist denkbar, dass die Speichereinheit 208 mit neuen Markern auffüllbar ist.

Die Versorgungseinheit 74 umfasst zumindest eine Mikrodosiereinheit 78. Die Mikrodosiereinheit 78 ist fluidtechnisch mit der Speichereinheit 208 verbunden. Die Mikrodosiereinheit 78 ist zur Förderung der Marker 76 vorgesehen. Die Mikrodosiereinheit 78 umfasst zumindest eine Mikropumpe (hier nicht weiter dargestellt).

Ferner weist die Versorgungseinheit 74 zumindest eine Versorgungsleitung 80 auf. Die Versorgungsleitung 80 ist fluidtechnisch mit einer Markerstation 114 der Messvorrichtung 14 verbunden. Die Versorgungsleitung 80 ist zur Aufbringung der Marker 76 auf das Versuchsband 52, insbesondere das Versuchsfeld 54, vorgesehen. Die Versorgungseinheit 74, insbesondere die Mikrodosiereinheit 78, ist von der Steuereinheit 34 zur Dosierung der Marker 76 ansteuerbar.

Die Kartuschenvorrichtung 44 weist zumindest eine Anordnungseinheit 210 auf (vgl. Fig. 5). Die Anordnungseinheit 210 ist zumindest zur Anordnung der Versorgungseinheit 74 an dem Kartuschengehäuse 46 vorgesehen. Die Anordnungseinheit 210 umfasst zumindest ein Anordnungselement 212. Das Anordnungselement 212 ist zur Anordnung der Speichereinheit 208 vorgesehen. Das Anordnungselement 212 ist als eine Aufnahmetasche ausgebildet. Die Speichereinheit 208 ist in dem Anordnungselement 212 anordnenbar, insbesondere einschiebbar. Das Anordnungselement 212 ist einstückig mit dem Kartuschengehäuse 46 ausgebildet. Ferner weist die Anordnungseinheit 210 zumindest ein weiteres Anordungselement auf. Der Übersichtlichkeit halber ist nur ein Anordnungselement 212 mit einem Bezugszeichen versehen. Das weitere Anordnungselement ist zu einer Anordnung der Mikrodosiereinheit 78 vorgesehen. Das weitere Anordnungselement ist als eine Aufnahmetasche ausgebildet. Das weitere Anordnungselement ist einstückig mit der Kartuschenvorrichtung 44 ausgebildet. Zudem weist die Anordnungseinheit 210 zumindest ein zusätzliches Anordnungselement 218 auf. Das zusätzliche Anordnungselement 218 ist zur Anordnung der Versorgungsleitung 80 vorgesehen. Das zusätzliche Anordnungselement 218 ist als eine Aufnahmeklammer ausgebildet. Das zusätzliche Anordnungselement 218 ist im Bereich eines Stegs 170 angeordnet.

Die Versuchseinheit 48 weist zumindest eine Spüleinheit 82 auf. Die Spüleinheit 82 ist zum Spülen zumindest eines Verbrauchsmaterials 50 der Versuchseinheit 48 vorgesehen. Die Spüleinheit 82 umfasst zumindest eine weitere Mikrodosiereinheit 79. Die weitere Mikrodosiereinheit 79 ist zumindest im Wesentlichen identisch zur Mikrodosiereinheit 78 ausgebildet. Ferner ist die Spüleinheit 82 zumindest teilweise im Bereich des Lagerabschnitts 86 angeordnet. Die Spüleinheit 82 ist an einer Außenseite des Kartuschengehäuses 46 angeordnet.

Die weitere Mikrodosiereinheit 79 ist fluidtechnisch mit einer Umgebungsluft verbunden. Die weitere Mikrodosiereinheit 79 ist dazu vorgesehen, einen Luftstrom zu erzeugen. Ferner weist die Spüleinheit 82 eine weitere Versorgungsleitung 84 auf. Die weitere Versorgungsleitung 84 ist fluidtechnisch mit der weiteren Mikrodosiereinheit 79 verbunden. Die weitere Versorgungsleitung 84 ist mit einer Spülstation 118 der Messvorrichtung 14 fluidtechnisch verbunden.

Ferner weist die Kartuschenvorrichtung 44 einen Versorgungsanschluss 220 auf. Der Versorgungsanschluss 220 bildet zumindest teilweise die Spülstation 118 und/oder die Markerstation 114 aus. Der Versorgungsanschluss 220 ist an einem abgerollten Versuchsbandabschnitt 56 des Versuchsbands 52 angeordnet. Der Versorgungsanschluss 220 ist an einem unbedeckten Versuchsbandabschnitt 56 des Versuchsbands 52 angeordnet. Der Versorgungsanschluss 220 ist als ein Trichter ausgebildet. Der Versorgungsanschluss 220 ist mit der Versorgungseinheit 74 und/oder der Spüleinheit 82, insbesondere über die jeweiligen Versorgungsleitungen 80, 84, verbunden. Der Versorgungsanschluss 220 ist im Bereich des Stegs 170 angeordnet. Die weitere Versorgungsleitung 84 ist entlang der Haupterstreckungsrichtung 70 des Versuchsbands 52 versetzt neben dem Versorgungsanschluss 220 angeordnet.

Die Messvorrichtung 14 umfasst mehrere Versuchsstationen 98, 100, 102. Das Versuchsband 52 wird entlang der Versuchsstationen 98, 100, 102 verfahren. Die Messvorrichtung 14 umfasst zumindest eine Dispergierstation 106. Ferner umfasst die Messvorrichtung 14 die Filterstation 110. Ferner umfasst die Messvorrichtung 14 die Markerstation 114. Ferner umfasst die Messvorrichtung 14 die Spülstation 118. Ferner umfasst die Messvorrichtung 14 eine Optikstation 122. Die Spülstation 118 ist in Haupterstreckungsrichtung 70 versetzt zur Filterstation 110 angeordnet. Die Markerstation 114 ist in Haupterstreckungsrichtung 70 versetzt zur Filterstation 110 angeordnet. Die Markerstation 114 und die Spülstation 118 sind zumindest teilweise einstückig ausgebildet. Die Optikstation 122 ist versetzt in Haupterstreckungsrichtung 70 zur Markerstation 114 angeordnet. Die Spülstation 118 und die Markerstation 114 sind zwischen der Filterstation 110 und der Optikstation 122 angeordnet.

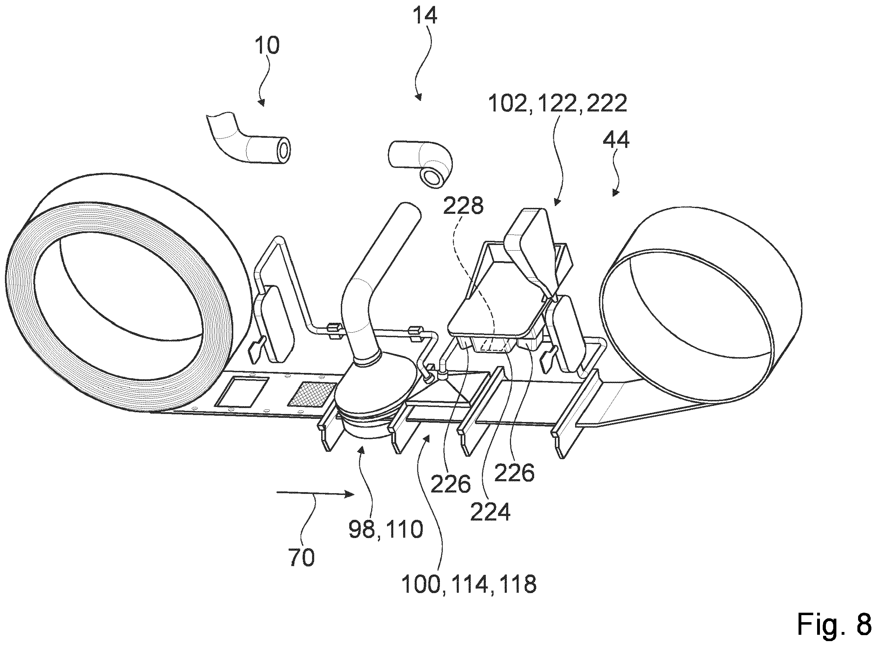

Fig. 8 zeigt einen Teil der Messvorrichtung 14 zusammen mit den Teilen der Kartuschenvorrichtung 44 und der Probenentnahmevorrichtung 10. Die Messvorrichtung 14 ist zu einer Messung einer Trinkwasserkontamination in der Trinkwasserleitung 12 durch Mikroorganismen 16 vorgesehen. Die Messvorrichtung 14 ist der Trinkwasserleitung 12 zugeordnet. Im vorliegenden Fall ist die Messvorrichtung 14 ortsfest der Trinkwasserleitung 12 zugeordnet. Die Messvorrichtung 14 ist fest mit der Trinkwasserleitung 12 verbunden. Im vorliegenden Fall verbindet die Probenentnahmevorrichtung 10 die Messvorrichtung 14 mit der Trinkwasserleitung 12. Die Messvorrichtung 14 ist dazu vorgesehen, eine von der Probenentnahmevorrichtung 10 bereitgestellte Trinkwasserprobe zur Messung der Trinkwasserkontamination zu verwenden. Die Messvorrichtung 14 umfasst eine Optikstation 122. Die Optikstation 122 ist zu einer Messung der Trinkwasserkontamination vorgesehen.

Die Messvorrichtung 14 umfasst zumindest eine Messeinheit 222. Die Messeinheit 222 bildet zumindest teilweise die Optikstation 122 aus. Die Messeinheit 222 umfasst zumindest einen optischen Sensor 224. Im vorliegenden Fall ist der optische Sensor 224 als ein Kamerasensor, insbesondere als ein CCD-Sensor, ausgebildet. Ferner umfasst die Messeinheit 222 zumindest eine Strahlungsquelle 226. Im vorliegenden Fall weist die Messeinheit 222 zwei Strahlungsquellen 226 auf. Ferner sind die Strahlungsquellen 226 als LEDs ausgebildet. Die Strahlungsquellen 226 sind zur Bereitstellung von UV-Licht vorgesehen. Die Strahlungsquellen 226 und der optische Sensor 224 sind derart angeordnet, dass sich eine Auflicht-Anordnung ergibt.

Eine von der Bestrahlung durch die Strahlungsquellen 226 ausgelöste Fluoreszenz der Marker 76 wird von dem optischen Sensor 224 der Messeinheit 222 detektiert. Ferner weist die Messeinheit 222 zumindest einen optischen Filter 228 auf. Der optische Filter 228 ist in einem Strahlengang des optischen Sensors 224 angeordnet. Der optische Filter 228 ist dazu vorgesehen, Strahlung in einem Spektralbereich der von den Strahlungsquellen 226 ausgesandten Strahlung zumindest teilweise, vorzugsweise zumindest zu einem Großteil, zu vermindern. Ferner ist der optische Filter 228 dazu vorgesehen, von den Markern 76 durch Anregung durch die Strahlung abgegebene Strahlung, insbesondere Fluoreszenz, zumindest im Wesentlichen unvermindert durchzulassen. Der optische Filter 228 ist als ein dichroitischer Filter 228 ausgebildet.

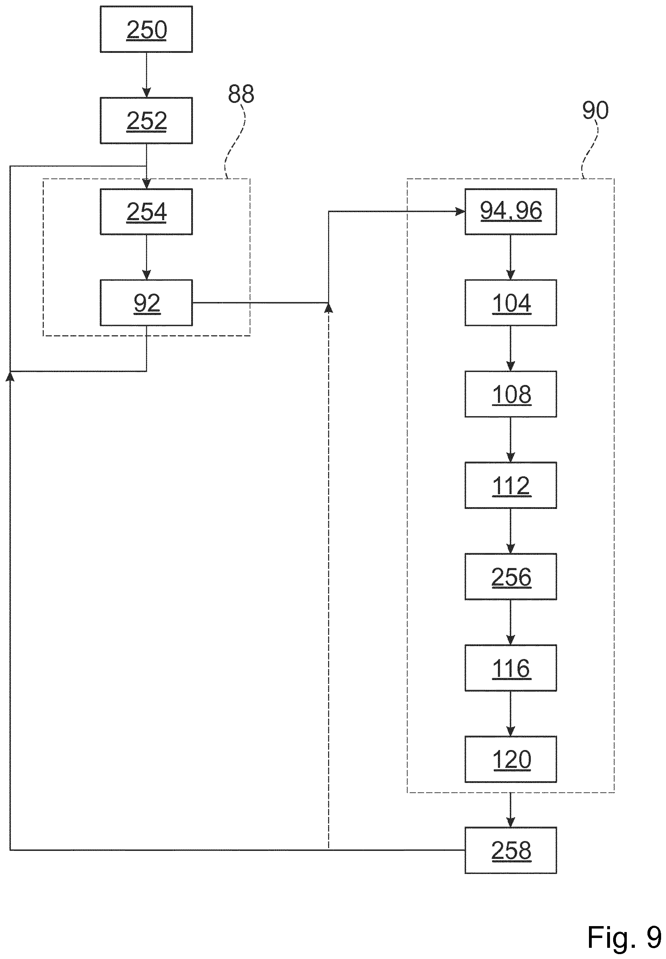

In Fig. 9. ist ein schematisches Ablaufdiagramm eines Verfahrens zum Betrieb zumindest der Analyseeinrichtung 42 dargestellt. Das Verfahren ist Teil des Betriebsprogramms, welches von der Steuereinheit 34 ausgeführt wird. Bei dem Verfahren handelt es sich um ein Verfahren zur Prognose und Messung einer Trinkwasserkontamination durch Mikroorganismen 16 in der Trinkwasserleitung 12. Im vorliegenden Fall wird das Verfahren ortsfest, also am Ort einer der Analyseeinrichtung 42 fest zugeordneten Trinkwasserleitung 12, durchgeführt. Alternativ könnte das Verfahren, insbesondere nach einer separat von dem Verfahren durchgeführten Probenentnahme, auch an einem von der Trinkwasserleitung 12 verschiedenen Ort durchgeführt werden.

Das Verfahren umfasst im vorliegenden Fall einen Verfahrensschritt 250. In dem Verfahrensschritt 250 wird die Kartuschenvorrichtung 44 in die Messvorrichtung 14 eingesetzt. Das Kartuschengehäuse 46 der Kartuschenvorrichtung 44 wird mit der Messvorrichtung 14 gekoppelt. Für den Fall, dass die Kartuschenvorrichtung 44 bereits mit der Messvorrichtung 14 gekoppelt ist, kann auf den Verfahrensschritt 250 verzichtet werden.

Das Verfahren zum Betrieb der Analyseeinrichtung 42 umfasst vorzugsweise zumindest ein Verfahren zum Betrieb einer austauschbaren Kartuschenvorrichtung, insbesondere der vorgehend beschriebenen Kartuschenvorrichtung 44. Insbesondere bildet das Verfahren zum Betrieb der austauschbaren Kartuschenvorrichtung 44 zumindest im Wesentlichen den Verfahrensschritt 250 des Verfahrens zum Betrieb der Analyseeinrichtung 42 aus. In zumindest einem Verfahrensschritt 250 des Verfahrens zum Betrieb der austauschbaren Kartuschenvorrichtung 44 für eine zumindest einer Trinkwasserleitung 12 insbesondere ortsfest zugeordnete Messvorrichtung 14 zur Messung einer Trinkwasserkontamination in der Trinkwasserleitung 12 durch Mikroorganismen 16, mit zumindest einem Kartuschengehäuse 46, in welchem zumindest teilweise zumindest eine Versuchseinheit 48 angeordnet ist, welche zumindest ein Verbrauchsmaterial 50 aufweist, das zur Durchführung zumindest eines Versuchs zur Messung der Trinkwasserkontamination vorgesehen ist, wird das Kartuschengehäuse 46 mit einer Messvorrichtung 14 gekoppelt.

Hinsichtlich weiterer Verfahrensschritte des Verfahrens zum Betrieb der austauschbaren Kartuschenvorrichtung 44 darf auf die vorhergehende Beschreibung der austauschbaren Kartuschenvorrichtung 44 verwiesen werden, da diese Beschreibung analog auch auf das Verfahren zu lesen ist und somit alle Merkmale hinsichtlich der austauschbaren Kartuschenvorrichtung 44 auch in Bezug auf das Verfahren zum Betrieb der austauschbaren Kartuschenvorrichtung 44 als offenbart gelten.

Das Verfahren umfasst zumindest einen weiteren Verfahrensschritt 252. In dem weiteren Verfahrensschritt 252 wird eine Analyse der Trinkwasserkontamination durch die Analyseeinrichtung 42 initiiert. Im vorliegenden Fall wird die Analyse durch die externe Einheit 128 initiiert. Dabei wird die Steuereinheit 34 der Analyseeinrichtung 42 von der externen Einheit 128 angesteuert, insbesondere ferngesteuert. Wird eine Analyse ortsfest an der Analyseeinrichtung 42 selbst initiiert und/oder ist eine automatische Initiierung, wie beispielsweise basierend auf einer Zeitschaltung, vorgesehen, so könnte auch auf die externe Einheit 128 verzichtet werden. Ferner kann bei einem kontinuierlichen Betrieb der Analyseeinrichtung 42 auf diesen Verfahrensschritt 252 verzichtet werden.

Das Verfahren umfasst zumindest einen Prognosevorgang 88. Unter einem "Vorgang", wie beispielsweise dem Prognosevorgang 88 und/oder dem Messvorgang 90, soll insbesondere zumindest ein Teil eines Verfahrens verstanden werden, welcher zumindest einen Verfahrensschritt, vorzugsweise zumindest zwei Verfahrensschritte und besonders bevorzugt mehrere Verfahrensschritte umfasst. Unter einem "Prognosevorgang" 88 soll insbesondere ein Vorgang verstanden werden, bei welchem eine Wahrscheinlichkeit einer Kontamination, insbesondere anhand der zumindest einen Kontaminationsrisikokenngröße, bestimmt wird, insbesondere nicht durch eine direkte Messung der die Trinkwasserkontamination verursachenden Mikroorganismen 16. Unter einem "Messvorgang" 90 soll insbesondere ein Vorgang verstanden werden, bei welchem eine Trinkwasserkontamination durch Mikroorganismen 16 direkt durch eine zumindest quantitative und vorzugsweise qualitative Messung der Mikroorganismen 16 durchgeführt wird. Der Prognosevorgang 88 ist zur Prognose der Trinkwasserkontamination vorgesehen. In dem Prognosevorgang 88 wird die Trinkwasserkontamination prognostiziert. In dem Prognosevorgang 88 wird eine Trinkwasserkontamination anhand zumindest einer Kontaminationsrisikokenngröße prognostiziert. Als Kontaminationsrisikokenngröße wird eine Temperatur des Trinkwassers 30 berücksichtigt. Im vorliegenden Fall wird in dem Prognosevorgang 88 die Trinkwasserkontamination anhand zumindest einer weiteren Kontaminationsrisikokenngröße prognostiziert. Die weitere Kontaminationsrisikokenngröße ist von der Kontaminationsrisikokenngröße verschieden. Als zumindest die eine weitere Kontaminationsrisikokenngröße wird ein Durchfluss des Trinkwassers 30 berücksichtigt. Der Prognosevorgang 88 wird wiederholt, insbesondere kontinuierlich, durchgeführt.

Der Prognosevorgang 88 umfasst zumindest einen Verfahrensschritt 254. In dem Verfahrensschritt 254 wird von der Sensoreinheit 20 zumindest eine Kontaminationsrisikokenngröße sensiert. Die Sensoreinheit 20 sensiert die Kontaminationsrisikokenngröße. Im vorliegenden Fall sensiert der Sensor 24, 150 die Kontaminationsrisikokenngröße. Ferner sensiert die Sensoreinheit 20 zumindest die weitere Kontaminationsrisikokenngröße. Im vorliegenden Fall sensiert der weitere Sensor 26, 152 der Sensoreinheit 20 die weitere Kontaminationsrisikokenngröße.

Der Prognosevorgang 88 umfasst zumindest einen weiteren Verfahrensschritt 92. In dem weiteren Verfahrensschritt 92 wird die Trinkwasserkontamination anhand des Kontaminationskennfelds 158 prognostiziert. Für den Fall, dass anhand einer prognostizierten Wahrscheinlichkeit der Trinkwasserkontamination ein Referenzwert überschritten wird, wird ein Messvorgang 90 initialisiert. Für den Fall, dass die prognostizierte Wahrscheinlichkeit der Trinkwasserkontamination den Referenzwert unterschreitet, wird der Prognosevorgang 88 wiederholt.

Das Verfahren umfasst zumindest einen Messvorgang 90. Der Messvorgang 90 ist zur Messung der Trinkwasserkontamination vorgesehen. In dem Messvorgang 90 wird eine tatsächliche Trinkwasserkontamination gemessen. Der Messvorgang 90 wird nach zumindest einer Durchführung des Prognosevorgangs 88 durchgeführt. Der Messvorgang 90 wird in Abhängigkeit von einer prognostizierten Trinkwasserkontamination durchgeführt.

Der Messvorgang 90 umfasst zumindest einen Verfahrensschritt 94. In dem Verfahrensschritt 94 wird das Versuchsband 52 entlang einer Haupterstreckungsrichtung 70 des Versuchsbands 52 bewegt. Das Versuchsband 52 wird zumindest abschnittsweise abgerollt. Gleichzeitig wird das Versuchsband 52 aufgerollt. In einem Lagerzustand des Versuchsbands 52 ist ein Versuchsfeld 54 des Versuchsbands 52 zumindest von einem weiteren Versuchsbandabschnitt 58 des Versuchsbands 52 bedeckt angeordnet. Wird das Versuchsband 52 bewegt, so wird der Versuchsbandabschnitt 58 des Versuchsbands 52, welcher das Versuchsfeld 54 vormals bedeckt hat, von diesem entfernt.

Der Messvorgang 90 umfasst zumindest einen weiteren Verfahrensschritt 96. In dem Verfahrensschritt 96 wird das Versuchsband 52 entlang verschiedener Versuchsstationen 98, 100, 102 bewegt. Zunächst wird das Versuchsband 52 so verfahren, dass ein Versuchsfeld 54 des Versuchsbands 52 im Bereich der Filterstation 110 angeordnet ist. Der Verfahrensschritt 96 erfolgt zumindest teilweise gleichzeitig mit dem Verfahrensschritt 94.

Der Messvorgang 90 weist zumindest einen weiteren Verfahrensschritt 104 auf. In dem Verfahrensschritt 104 wird eine Trinkwasserprobe des Trinkwassers 30, welche von der Probenentnahmevorrichtung 10 entnommen wurde, an einer Dispergierstation 106 dispergiert.

Der Messvorgang 90 umfasst zumindest einen weiteren Verfahrensschritt 108. In dem weiteren Verfahrensschritt 108 wird die Trinkwasserprobe des Trinkwassers 30 an einer Filterstation 110 mittels zumindest eines Versuchsfelds 54 eines Versuchsbands 52 mikrofiltriert. Die kontaminierenden Mikroorganismen 16 werden auf dem Versuchsfeld 54 zumindest teilweise zurückgehalten.

Der Messvorgang 90 umfasst zumindest einen weiteren Verfahrensschritt 112. In dem weiteren Verfahrensschritt 112 wird das Versuchsband 52 weiter in Haupterstreckungsrichtung 70 bewegt, bis dieses im Bereich der Markerstation 114 angeordnet ist. In dem weiteren Verfahrensschritt 112 werden an der Markerstation 114 Mikroorganismen 16 mit den Markern 76 versehen. Die Marker 76 spezifizieren zumindest eine Mikroorganismenart der Mikroorganismen 16 intrinsisch.

Ferner umfasst der Messvorgang 90 zumindest einen weiteren Verfahrensschritt 256. In dem weiteren Verfahrensschritt 256 wird die Filterstation 110 gereinigt. Durch Bewegen des Versuchsbands 52 wird ein Reinigungsfeld 66 im Bereich der Filterstation 110 angeordnet. Dies tritt aufgrund der Anordnung des Reinigungsfelds 66 und des Versuchsfelds 54 zueinander sowie der Filterstation 110 und der Markerstation 114 automatisch dann auf, wenn das Versuchsfeld 54 im Bereich der Markerstation 114 angeordnet ist. Die Filterstation 110 wird durch Spülen mit Trinkwasser 30 gereinigt.

Der Messvorgang 90 umfasst zumindest einen weiteren Verfahrensschritt 116. In dem weiteren Verfahrensschritt 116 werden an der Spülstation 118 überschüssige Marker 76 ausgespült. Dies geschieht durch die von der Spüleinheit 82 zur Verfügung gestellte Umgebungsluft. An der Spülstation 118 werden überschüssige Marker 76, die an der Filterstation 110 mittels eines flüssigen Fluids, insbesondere mittels Wasser, nicht ausgespült wurden und/oder ein überschüssiges Fluid, ausgepült. Im vorliegenden Fall sind die Markerstation 114 und die Spülstation 118 identisch angeordnet, so dass das Versuchsband 52 zur Durchführung des Verfahrensschritts 116 nicht weiterbewegt werden muss.

Der Messvorgang 90 umfasst zumindest einen weiteren Verfahrensschritt 120. In dem weiteren Verfahrensschritt 120 werden an einer Optikstation 122 die Mikroorganismen 16 optisch detektiert. Die Detektion wird von der Messeinheit 222 durchgeführt. Dabei wird ein Abbild der Mikroorganismen 16 erzeugt. Das Abbild weist dabei eine Auflösung einer Rasterbreite von höchstens 5 µm auf. Ferner wird in dem weiteren Verfahrensschritt 120 an der Optikstation 122 eine Trinkwasserkontamination anhand einer Quantität der Mikroorganismen 16 bestimmt. Vorzugsweise werden einzelne Punkte des Abbilds mittels eines Algorithmus analysiert und quantitativ ausgezählt. Für den Fall, dass eine Trinkwasserkontamination festgestellt wird, kann ein Benutzer darüber informiert werden, beispielsweise über die externe Einheit 128.

Ferner umfasst das Verfahren einen weiteren Verfahrensschritt 258. In dem weiteren Verfahrensschritt 258 wird die Entnahmeeinheit 18 durch die Desinfektionseinheit 38 desinfiziert. Anschließend kann der Prognosevorgang 88 wiederholt werden. Alternativ oder zusätzlich könnte auch der Messvorgang 90 wiederholt werden, insbesondere für den Fall, dass eine Messung der Kontamination wesentlich von einer prognostizierten Kontamination abweicht.

Hinsichtlich weiterer Verfahrensschritte des Verfahrens zum Betrieb der Analyseeinrichtung 42 darf auf die vorhergehende Beschreibung der Analyseeinrichtung 42 verwiesen werden, da diese Beschreibung analog auch auf das Verfahren zu lesen ist und somit alle Merkmale hinsichtlich der Analyseeinrichtung 42 auch in Bezug auf das Verfahren zum Betrieb der Analyseeinrichtung 42 als offenbart gelten.

### Bezugszeichen

- 10: Probenentnahmevorrichtung
- 12: Trinkwasserleitung
- 14: Messvorrichtung
- 16: Mikroorganismus
- 18: Entnahmeeinheit
- 20: Sensoreinheit
- 22: Anschlusskopf
- 24: Temperatursensor
- 26: Durchflusssensor
- 28: Dispergiereinheit
- 30: Trinkwasser
- 32: Entkopplungseinheit
- 34: Steuereinheit
- 36: Filtereinheit
- 38: Desinfektionseinheit
- 40: Temperierelement
- 42: Analyseeinrichtung
- 44: Kartuschenvorrichtung
- 46: Kartuschengehäuse
- 48: Versuchseinheit
- 50: Verbrauchsmaterial
- 51: Verbrauchsmaterial
- 52: Versuchsband
- 54: Versuchsfeld
- 56: Versuchsbandabschnitt
- 58: Versuchsbandabschnitt
- 60: Schichtaufbau
- 62: Filter
- 64: Senke
- 66: Reinigungsfeld
- 68: Reinigungsfeld
- 70: Haupterstreckungsrichtung
- 72: Dichteinheit
- 74: Versorgungseinheit
- 76: Marker
- 78: Mikrodosiereinheit
- 79: Mikrodosiereinheit
- 80: Versorgungsleitung
- 82: Spüleinheit
- 84: Versorgungsleitung
- 86: Lagerabschnitt
- 88: Prognosevorgang
- 90: Messvorgang
- 92: Verfahrensschritt
- 94: Verfahrensschritt
- 96: Verfahrensschritt
- 98: Versuchsstation
- 100: Versuchsstation
- 102: Versuchsstation
- 104: Verfahrensschritt
- 106: Dispergierstation
- 108: Verfahrensschritt
- 110: Filterstation
- 112: Verfahrensschritt
- 114: Markerstation
- 116: Verfahrensschritt
- 118: Spülstation
- 120: Verfahrensschritt
- 122: Optikstation
- 124: Armatur
- 126: Waschbeckengarnitur
- 128: Externe Einheit
- 130: Lagerabschnitt
- 132: Anschluss
- 134: Anschluss
- 136: Anschluss
- 138: Entnahmeleitung
- 140: Ventileinheit
- 142: Ventil
- 144: Ventil
- 146: Filterelement
- 148: Stützschicht
- 150: Sensor
- 152: Sensor
- 154: Entkopplungselement
- 156: Sensoreinheit
- 158: Kontaminationskennfeld
- 160: Abszissenachse
- 162: Ordinatenachse
- 164: Wahrscheinlichkeitsbereich
- 166: Wahrscheinlichkeitsbereich
- 168: Wahrscheinlichkeitsbereich
- 170: Steg
- 172: Behälter
- 174: Deckel
- 176: Koppeleinheit
- 178: Rastelement
- 180: Betätigungselement
- 182: Führungselement
- 184: Abrollspule
- 186: Aufrollspule
- 188: Achsaufnahme
- 190: Achsaufnahme
- 192: Antriebsachse
- 194: Antriebsachse
- 196: Filterschicht
- 198: Trägerschicht
- 200: Trägerschicht
- 202: Trennfeld
- 204: Ausnehmung
- 206: Dichtung
- 208: Speichereinheit
- 210: Anordnungseinheit
- 212: Anordnungselement
- 214: Trinkwasserprobenanschluss
- 216: Trinkwasserprobenauslass
- 218: Anordnungselement
- 220: Versorgungsanschluss
- 222: Messeinheit
- 224: Optischer Sensor
- 226: Strahlungsquelle
- 228: Optischer Filter
- 250: Verfahrensschritt
- 252: Verfahrensschritt
- 254: Verfahrensschritt
- 256: Verfahrensschritt
- 258: Verfahrensschritt

## Patentansprüche

1. Austauschbare Kartuschenvorrichtung für eine zumindest einer Trinkwasserleitung (12) insbesondere ortsfest zugeordnete Messvorrichtung (14) zur Messung einer Trinkwasserkontamination in der Trinkwasserleitung (12) durch Mikroorganismen (16), insbesondere durch Legionellen, mit zumindest einem Kartuschengehäuse (46), welches mit der Messvorrichtung (14) koppelbar ist, und mit zumindest einer Versuchseinheit (48), welche zumindest teilweise in dem Kartuschengehäuse (46) angeordnet ist, wobei die Versuchseinheit (48) zumindest ein Verbrauchsmaterial (50) aufweist, das zur Durchführung zumindest eines Versuchs zur Messung der Trinkwasserkontamination vorgesehen ist, wobei die Versuchseinheit (48) zumindest eine Versorgungseinheit (74) zur Bereitstellung von Markern (76) zur Markierung der Mikroorganismen (16) umfasst, **dadurch gekennzeichnet, dass** die Versorgungseinheit (74) zumindest eine Mikrodosiereinheit (78) umfasst.

2. Austauschbare Kartuschenvorrichtung nach Anspruch 1, **dadurch gekennzeichnet, dass** die Versuchseinheit (48) zumindest ein Versuchsband (52) umfasst, welches zumindest ein Versuchsfeld (54) aufweist, welches zur Durchführung des Versuchs vorgesehen ist.

3. Austauschbare Kartuschenvorrichtung nach Anspruch 2, **dadurch gekennzeichnet, dass** in zumindest einem Lagerzustand des Versuchsbands (52) zumindest ein Versuchsbandabschnitt (56) des Versuchsbands (52) zumindest teilweise von zumindest einem weiteren Versuchsbandabschnitt (58) des Versuchsbands (52) bedeckt ist.

4. Austauschbare Kartuschenvorrichtung nach Anspruch 2 oder 3, **dadurch gekennzeichnet, dass** in zumindest einem Lagerzustand des Versuchsbands (52) das Versuchsband (52) zumindest abschnittsweise aufgerollt gelagert ist.

5. Austauschbare Kartuschenvorrichtung nach einem der Ansprüche 2 bis 4, **dadurch gekennzeichnet, dass** das Versuchsband (52) einen Schichtaufbau (60), insbesondere in Form einer Sandwichbauweise, aufweist.

6. Austauschbare Kartuschenvorrichtung nach einem der Ansprüche 2 bis 5, **dadurch gekennzeichnet, dass** das Versuchsfeld (54) zumindest einen Filter (62) umfasst, welcher zur Abscheidung von Mikroorganismen (16) aus Trinkwasser (30) vorgesehen ist.

7. Austauschbare Kartuschenvorrichtung nach einem der Ansprüche 2 bis 6, **dadurch gekennzeichnet, dass** das Versuchsfeld (54) eine Senke (64) aufweist.

8. Austauschbare Kartuschenvorrichtung nach einem der Ansprüche 2 bis 7, **dadurch gekennzeichnet, dass** das Versuchsband (52) zumindest ein Reinigungsfeld (66) aufweist, welches zur Spülung mit Trinkwasser (30) vorgesehen ist.

9. Austauschbare Kartuschenvorrichtung nach Anspruch 8, **dadurch gekennzeichnet, dass** das Reinigungsfeld (66) zumindest eine Ausnehmung (204) aufweist.

10. Austauschbare Kartuschenvorrichtung zumindest nach Anspruch 2 und Anspruch 9, **dadurch gekennzeichnet, dass** das Versuchsfeld (54) und das Reinigungsfeld (66) entlang einer Haupterstreckungsrichtung (70) des Versuchsbands (52) versetzt zueinander angeordnet sind.

11. Austauschbare Kartuschenvorrichtung nach einem der vorhergehenden Ansprüche, **gekennzeichnet durch** zumindest eine Dichteinheit (72), welche die Versuchseinheit (48) zumindest abschnittsweise fluiddicht abdichtet.

12. Austauschbare Kartuschenvorrichtung zumindest nach Anspruch 2 und Anspruch 11, **dadurch gekennzeichnet, dass** die Dichteinheit (72) dichtend an zumindest einem Versuchsbandabschnitt (56) anliegt.

13. Austauschbare Kartuschenvorrichtung nach einem der vorhergehenden Ansprüche, **dadurch gekennzeichnet, dass** die Versorgungseinheit (74) zumindest eine Versorgungsleitung (80) umfasst, welche zur Aufbringung der Marker (76) vorgesehen ist.

14. Austauschbare Kartuschenvorrichtung nach einem der vorhergehenden Ansprüche, **dadurch gekennzeichnet, dass** die Versuchseinheit (48) zumindest eine Spüleinheit (82) umfasst, welche zum Spülen zumindest eines Verbrauchsmaterials (50) der Versuchseinheit (48) vorgesehen ist.

15. Austauschbare Kartuschenvorrichtung nach einem der Ansprüche 2 bis 14, **dadurch gekennzeichnet, dass** die Versuchseinheit (48) zumindest einen Versorgungsanschluss (220) aufweist, welcher an einem abgerollten Versuchsbandabschnitt (56) des Versuchsbands (52) angeordnet ist.

16. Austauschbare Kartuschenvorrichtung nach den Ansprüchen 2, 13 und 15, **dadurch gekennzeichnet, dass** die Versorgungseinheit (74) zumindest eine weitere Versorgungsleitung (84) aufweist, welche entlang einer Haupterstreckungsrichtung (70) des Versuchsbands (52) versetzt neben dem Versorgungsanschluss (220) angeordnet ist.

17. Verfahren zum Betrieb einer austauschbaren Kartuschenvorrichtung (44) nach einem der vorhergehenden Ansprüche, für eine zumindest einer Trinkwasserleitung (12) insbesondere ortsfest zugeordnete Messvorrichtung (14) zur Messung einer Trinkwasserkontamination in der Trinkwasserleitung (12) durch Mikroorganismen (16), mit zumindest einem Kartuschengehäuse (46), in welchem zumindest teilweise zumindest eine Versuchseinheit (48) angeordnet ist, welche zumindest ein Verbrauchsmaterial (50) aufweist, das zur Durchführung zumindest eines Versuchs zur Messung der Trinkwasserkontamination vorgesehen ist, **dadurch gekennzeichnet, dass** in zumindest einem Verfahrensschritt (250) das Kartuschengehäuse (46) mit einer Messvorrichtung (14) gekoppelt wird.

18. Analyseeinrichtung (42) mit zumindest einer zumindest einer Trinkwasserleitung (12) insbesondere ortsfest zugeordneten Messvorrichtung (14) zur Messung einer Trinkwasserkontamination in der Trinkwasserleitung (12) durch Mikroorganismen (16) und mit zumindest einer austauschbaren Kartuschenvorrichtung (44) nach einem der Ansprüche 1 bis 16.

## Claims

1. Exchangeable cartridge device for a measuring apparatus (14) assigned, in particular stationarily, to at least one drinking water conduit (12) for a measurement of a drinking water contamination in the drinking water conduit (12) by microorganisms (16), in particular by legionellae,
with at least one cartridge housing (46) which can be coupled with the measuring apparatus (14) and
with at least one test unit (48) which is arranged at least partially in the cartridge housing (46),
the test unit (48) comprising at least one consumable material (50) configured for carrying out at least one test for measuring the drinking water contamination, the test unit (48) comprising at least one supply unit (74) to provide markers (76) for a marking of the microorganisms (16),
**characterised in that** the supply unit (74) comprises at least one microdosing unit (78).

2. Exchangeable cartridge device according to claim 1,
**characterised in that** the test unit (48) comprises at least one test strip (52) having at least one test zone (54) that is configured for carrying out the test.

3. Exchangeable cartridge device according to claim 2,
**characterised in that** in at least one storage state of the test strip (52), at least one test strip section (56) of the test strip (52) is covered at least partly by at least one further test strip section (58) of the test strip (52).

4. Exchangeable cartridge device according to claim 2 or 3,
**characterised in that** in at least one storage state of the test strip (52), the test strip (52) is stored such that it is at least partly rolled up.

5. Exchangeable cartridge device according to one of claims 2 to 4,
**characterised in that** the test strip (52) has a layer structure (60), in particular in the form of a sandwich construction.

6. Exchangeable cartridge device according to one of claims 2 to 5,
**characterised in that** the test zone (54) comprises at least one filter (62) that is configured for a precipitation of microorganisms (16) from drinking water (30).

7. Exchangeable cartridge device according to one of claims 2 to 6,
**characterised in that** the test zone (54) has a deepening (64).

8. Exchangeable cartridge device according to one of claims 2 to 7,
**characterised in that** the test strip (52) has at least one cleaning zone (66) that is configured for a rinsing with drinking water (30).

9. Exchangeable cartridge device according to claim 8,
**characterised in that** the cleaning zone (66) comprises at least one recess (204).

10. Exchangeable cartridge device at least according to claim 2 and claim 9,
**characterised in that** the test zone (54) and the cleaning zone (66) are arranged offset from each other along a main extension direction (70) of the test strip (52).

11. Exchangeable cartridge device according to one of the preceding claims,
**characterised by** at least one sealing unit (72) which seals the test unit (48) at least section-wise in a fluid-tight manner.

12. Exchangeable cartridge device at least according to claim 2 and claim 11,
**characterised in that** the sealing unit (72) is sealingly adjacent to at least one test strip section (56).

13. Exchangeable cartridge device according to one of the preceding claims,
**characterised in that** the supply unit (74) comprises at least one supply conduit (80) which is configured for an application of the markers (76).

14. Exchangeable cartridge device according to one of the preceding claims,
**characterised in that** the test unit (48) comprises at least one rinsing unit (82), which is configured for a rinsing of at least one consumable material (50) of the test unit (48).

15. Exchangeable cartridge device according to one of claims 2 to 14,
**characterised in that** the test unit (48) comprises at least one supply connection (220), which is arranged on a rolled-off test strip section (56) of the test strip (52).

16. Exchangeable cartridge device according to claims 2, 13 and 15,
**characterised in that** the supply unit (74) comprises at least one further supply conduit (84), which is arranged side by side with the supply connection (220), offset along a main extension direction (70) of the test strip (52).

17. Method for operating an exchangeable cartridge device (44) according to one of the preceding claims
for a measuring apparatus (14) assigned, in particular stationarily, to at least one drinking water conduit (12) for a measurement of a drinking water contamination in the drinking water conduit (12) by microorganisms (16),
with at least one cartridge housing (46) in which at least one test unit (48) is arranged at least partly, said test unit (48) comprising at least one consumable material (50) that is configured for carrying out at least one test for measuring the drinking water contamination,
**characterised in that** in at least one method step (250) the cartridge housing (46) is coupled with a measuring apparatus (14).

18. Analysis apparatus (42)
with at least one measuring apparatus (14) assigned, in particular stationarily, to at least one drinking water conduit (12) for a measurement of a drinking water contamination in the drinking water conduit (12) by microorganisms (16), and with at least one exchangeable cartridge device (44) according to one of claims 1 to 16.

## Revendications

1. Dispositif de cartouche interchangeable pour un appareil de mesure (14) alloué, en particulier de façon stationnaire, à au moins une conduite d'eau potable (12), pour mesurer une contamination d'eau potable dans la conduite d'eau potable (12) par des microorganismes (16), en particulier par des légionelles,
avec au moins un boîtier de cartouche (46) qui peut être couplé avec l'appareil de mesure (14) et
avec au moins une unité expérimentale (48) disposée au moins partiellement dans le boîtier de cartouche (46),
l'unité expérimentale (48) comprenant au moins un matériau de consommation (50) prévu pour l'exécution d'au moins une expérience pour mesurer la contamination d'eau potable,
l'unité expérimentale (48) comprenant au moins une unité d'approvisionnement (74) afin de fournir des marqueurs (76) pour marquer les microorganismes (16),
**caractérisée en ce que** l'unité d'approvisionnement (74) comprend au moins une unité à microdosage (78).

2. Dispositif de cartouche interchangeable selon la revendication 1,
**caractérisé en ce que** l'unité expérimentale (48) comprend au moins une bande de test (52) ayant au moins une zone expérimentale (54) qui est prévue pour l'exécution de l'expérience.

3. Dispositif de cartouche interchangeable selon la revendication 2,
**caractérisé en ce que** dans au moins un état de stockage de la bande de test (52) au moins une section de bande de test (56) de la bande de test (52) est au moins partiellement couverte d'au moins une autre section de bande de test (58) de la bande de test (52).

4. Dispositif de cartouche interchangeable selon la revendication 2 ou 3,
**caractérisé en ce que** dans au moins un état de stockage de la bande de test (52) la bande de test (52) est stockée de manière à être roulée au moins par sections.

5. Dispositif de cartouche interchangeable selon l'une des revendications 2 à 4,
**caractérisé en ce que** la bande de test (52) présente une structure en couches (60), en particulier dans la forme d'une construction en sandwich.

6. Dispositif de cartouche interchangeable selon l'une des revendications 2 à 5,
**caractérisé en ce que** la zone expérimentale (54) comprend au moins un filtre (62) prévu pour la précipitation des microorganismes (16) de l'eau potable (30).

7. Dispositif de cartouche interchangeable selon l'une des revendications 2 à 6,
**caractérisé en ce que** la zone expérimentale (54) comprend une dépression (64).

8. Dispositif de cartouche interchangeable selon l'une des revendications 2 à 7,
**caractérisé en ce que** la bande de test (52) comprend au moins une zone de nettoyage (66) prévue pour le rinçage avec de l'eau potable (30).

9. Dispositif de cartouche interchangeable selon la revendication 8,
**caractérisé en ce que** la zone de nettoyage (66) comprend au moins un renfoncement (204).

10. Dispositif de cartouche interchangeable au moins selon la revendication 2 et la revendication 9,
**caractérisé en ce que** la zone expérimentale (54) et la zone de nettoyage (66) sont disposées en décalé l'une par rapport à l'autre le long d'une direction d'étendue principale (70) de la bande de test (52).

11. Dispositif de cartouche interchangeable selon l'une des revendications précédentes,
**caractérisé par** au moins une unité d'étanchéité (72) scellant, au moins par sections, l'unité expérimentale (48) de façon étanche aux fluides.

12. Dispositif de cartouche interchangeable au moins selon la revendication 2 et la revendication 11,
**caractérisé en ce que** l'unité d'étanchéité (72) est adjacente à au moins une section de bande de test (56) en scellant.

13. Dispositif de cartouche interchangeable selon l'une des revendications précédentes,
**caractérisé en ce que** l'unité d'approvisionnement (74) comprend au moins une conduite d'approvisionnement (80) prévue pour l'application des marqueurs (76).

14. Dispositif de cartouche interchangeable selon l'une des revendications précédentes,
**caractérisé en ce que** l'unité expérimentale (48) comprend au moins une unité de rinçage (82) prévue pour le rinçage d'au moins un matériau de consommation (50) de l'unité expérimentale (48).

15. Dispositif de cartouche interchangeable selon l'une des revendications 2 à 14,
**caractérisé en ce que** l'unité expérimentale (48) comprend au moins une connexion d'approvisionnement (220) disposée sur une section de bande de test (56) déroulée de la bande de test (52).

16. Dispositif de cartouche interchangeable selon les revendications 2, 13 et 15,
**caractérisé en ce que** l'unité d'approvisionnement (74) comprend au moins une autre conduite d'approvisionnement (84) disposée en décalé le long d'une direction d'étendue principale (70) de la bande de test (52) à côté de la connexion d'approvisionnement (220).

17. Procédé pour le fonctionnement d'un dispositif de cartouche interchangeable (44) selon l'une des revendications précédentes,
pour au moins un appareil de mesure (14) alloué, en particulier de façon stationnaire, à au moins une conduite d'eau potable (12) pour mesurer une contamination d'eau potable dans la conduite d'eau potable (12) par des microorganismes (16),
avec au moins un boîtier de cartouche (46) dans lequel au moins une unité expérimentale (48) est disposée au moins partiellement,
ladite unité expérimentale (48) comprenant au moins un matériau de consommation (50) prévu pour l'exécution d'au moins une expérience pour mesurer la contamination d'eau potable,
**caractérisé en ce que** dans au moins une étape de procédé (250) le boîtier de cartouche (46) est couplé avec un appareil de mesure (14).

18. Appareil d'analyse (42)
avec au moins un appareil de mesure (14) alloué, en particulier de façon stationnaire, à au moins une conduite d'eau potable (12) pour mesurer une contamination d'eau potable dans la conduite d'eau potable (12) par des microorganismes (16) et
avec au moins un dispositif de cartouche interchangeable (44) selon l'une des revendications 1 à 16.
